# EUROPÄISCHE PATENTSCHRIFT

(11) **EP 1 966 212 B1**
(45) Veröffentlichungstag und Bekanntmachung des Hinweises auf die Patenterteilung: **13.05.2015**
(21) Anmeldenummer: 06829158.2
(22) Anmeldetag: 28.11.2006
(51) Int. Cl.: C07D 487/04, A61K 31/5517, A61P 35/00

(54) **DIAZEPINONE**
DIAZEPINONES
DIAZÉPINONES

(30) Priorität: 22.12.2005 DE 102005061655
(43) Veröffentlichungstag der Anmeldung: 10.09.2008
(73) Patentinhaber: Merck Patent GmbH, 64293 Darmstadt (DE)
(72) Erfinder: SCHULTZ, Melanie, 64287 Darmstadt (DE); BURGDORF, Lars, Thore, 60389 Frankfurt am Main (DE); FINSINGER, Dirk, 64291 Darmstadt (DE); BLAUKAT, Andree, 64367 Muehltal (DE); GREINER, Hartmut, 64331 Weiterstadt (DE); ESDAR, Christina, 55128 Mainz (DE)
(86) Internationale Anmeldenummer: PCT/EP2006/011411
(87) Internationale Veröffentlichungsnummer: WO 2007/079826

(56) Entgegenhaltungen:
- WO-A-81/00568
- WO-A-2004/076424
- US-A- 3 872 122
- PALANKI MOORTHY S S ET AL: "Synthesis and structure-activity relationship studies of conformationally restricted analogs of 2-chloro-4-trifluoromethylpyri midine-5-(N-(3',5'-bis(trifluoromethyl)phe nyl))carboxamide" MEDICINAL CHEMISTRY RESEARCH, Bd. 10, Nr. 1, 2000, Seiten 19-29, XP009081250 ISSN: 1054-2523
- DLUGOSZ A: "Synthesis and biological activity of pyrimidobenzodiazepine derivatives. New ring systems: triazolo- and tetrazolo-pyrimido-benzodiazepines." DIE PHARMAZIE MAR 1995, Bd. 50, Nr. 3, März 1995 (1995-03), Seiten 180-182, XP001249180 ISSN: 0031-7144

## Beschreibung

Die Erfindung betrifft Verbindungen der Formel AV 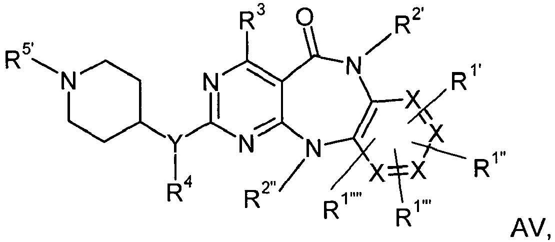 worin
- R^{1'}, R^{1"}, R^{1'"}, R^{1""}, R³, R⁴, R^{5'},: jeweils unabhängig voneinander H, A, R⁶, Ar, OR⁶, SR⁶, OAr, SAr, N(R⁶)₂, NHAr, Hal, NO₂, CN, (CH₂)ₘCOOR⁶, (CH₂)ₘCOOAr, (CH₂)ₘCON(R⁶)₂, (CH₂)ₘCONAAr, COA, COR⁶, COAr, S(O)ₘA, S(O)ₘAr, NACOA, NACOAr, NASO₂A, NASO₂Ar, NHCOA, NHCOAr, NHCON(R⁶)₂, NHCONHA, NHCONHAr, SO₂N(R⁶)₂, SO₂NAAr, M(CH₂)ₙN(R⁶)₂, M(CH₂)ₙNAR⁶, M(CH₂)ₙNA₂, M(CH₂)ₙ(R⁶)ₙ, M(CH₂)ₙ(R⁶)ₙ, M(CH₂)ₙ(R⁶)ₙ, M(CH₂)ₙ(R⁶)ₙ, M(CH₂)ₙ-oxopiperazin, M(CH₂)ₙ-oxomorpholin, M(CH₂)ₙ-oxopyrrolidin,
M(CH₂)ₙC(CH₃)ₙ(CH₂)ₙN(R⁶)₂, M(CH₂)ₙM(R⁶)ₙSOₘA, M(CH₂)ₙM(R⁶)ₙSOₘM(R⁶)ₙ, M(CH₂)ₙM(R⁶)ₙSOₘAr, (CH₂)ₙM(R⁶)ₙSOₘA, (CH₂)ₙM(R⁶)ₙSOₘM(R⁶)ₙ, (CH₂)ₙM(R⁶)ₙSOₘAr, M(CH₂)ₙSOₘA, M(CH₂)ₙSOₘN(R⁶)ₙA, M(CH₂)ₙSOₘAr, (CH₂)ₙSOₘA, (CH₂)ₙSOₘM(R⁶)ₙ, (CH₂)ₙSOₘAr,
wobei zwei benachbart stehende Reste R^{1'}, R^{1"}, R^{1'"} oder R^{1""}, miteinander einen gesättigten oder ungesättigten, optional ein- oder zweifach durch M substituierten, fünf- oder sechsgliedrigen Carbo- oder Heterozyklus bilden können,
- R^{2'}, R^{2"}: jeweils unabhängig voneinander R⁶,
- R⁶: H, Hal, OH, CN, NH₂, NO₂, SO₂, unverzweigtes oder verzweigtes Alkyl mit 1-4 C-Atomen, worin eine CH₂-Gruppe durch ein O oder S-Atom und/oder durch eine NH, NA, CONH, NHCO oder -CH=CH- Gruppe und/oder auch 1-4 H-Atome durch Hal ersetzt sein können, und worin eine CH₃-Gruppe durch Hal, OH, CN, NH₂, NHR₇, NR⁷₂, NO₂ oder SO₂ ersetzt sein kann, wobei R⁷ = Methyl oder Ethyl bedeutet, wobei zwei Reste R⁶ zusammen mit dem Atom, mit dem sie veknüpft sind, einen gesättigten oder ungesättigten fünf- oder sechsgliedrigen Carbo- oder Heterozyklus bilden können,
- n: 0, 1, 2, 3, 4 oder 5,
- m: 0, 1 oder 2,
- A: unverzweigtes, verzweigtes oder cyclisches Alkyl mit 1-14 C-Atomen, worin eine oder zwei CH₂-Gruppen durch ein O-oder S-Atom und/oder durch eine NH, CONH, NHCO, CO oder -CH=CH- Gruppe und/oder auch 1-7 H-Atome durch Hal ersetzt sein können, und worin eine oder zwei CH₃-Gruppen durch R⁶ ersetzt sein können,
- Ar: einen ein- oder zweikernigen aromatischen Homo- oder Heterocyclus mit 1 bis 4 N-, O- und/oder S-Atomen und 5 bis 10 Gerüstatomen, der unsubstituiert oder ein-, zwei- oder dreifach durch Carbonylsauerstoff, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ und/oder S(O)ₘA substituiert sein kann,
- Hal: F, Cl, Br oder I,
- X: CR¹ ist, worin R¹ stellvertretend für einen der Reste R^{1'}, R^{1"}, R^{1"'} oder R^{1""} steht,
- Y: NR⁴, O oder S, und
- M: NH, O, S
bedeuten,
sowie ihre pharmazeutisch unbedenklichen Salze, Solvate und Stereoisomere, einschließlich deren Mischungen in allen Verhältnissen.

Es wurde gefunden, dass die Verbindungen der Formel AV die Signaltransduktion, die durch Kinasen vermittelt wird, hemmen, regulieren und/oder modulieren können. Insbesondere eignen sich die erfindungsgemäßen Verbindungen als Inhibitoren von Kinasen. So können erfindungsgemäße Medikamente und pharmazeutische Zusammensetzungen wirksam zur Behandlung von Krankheiten eingesetzt werden, die durch Kinasen und/oder durch kinase-vermittelte Signaltransduktion verursacht, vermittelt und/oder propagiert werden. Somit eignen sich die erfindungsgemäßen Verbindungen zur Behandlung und Prophylaxe von Krebs, Tumorwachstum, Arteriosklerose, diabetischer Retinopathie, Entzündungserkrankungen, Psoriasis und dergleichen bei Säugetieren.

### Hintergrund der Erfindung

Krebs ist eine Krankheit, deren Ursachen unter anderem in einer gestörten Signaltransduktion zu sehen sind. Insbesondere deregulierte Signaltransduktion über Kinasen spielt eine zentrale Rolle bei der Entstehung, beim Wachstum und der Ausbreitung von Krebs (Blume-Jensen, P. und T. Hunter, Nature 411: 355-365, 2001; Hanahan D. und R. A. Weinberg, Cell 100:57-70, 2000). Verschiedene Rezeptor-Kinasen und cytoplasmatische Kinasen sowie die an sie bindenden Wachstumsfaktoren können so an deregulierter Apoptose, Gewebeinvasion, Metastasierung und allgemein an Signaltransduktionsmechanismen, die zu Krebs führen, beteiligt sein.

Wie bereits erwähnt, ist einer der Hauptmechanismen, durch den die Zellregulation bewirkt wird, die Transduktion der extrazellulären Signale über die Membran, die wiederum biochemische Wege in der Zelle modulieren.

Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und beeinflussen sich häufig gegenseitig, wie aus dem Vorliegen vieler Proteinkinasen wie auch Phosphatasen hervorgeht. Phosphorylierung von Proteinen tritt bei Serin-, Threonin- oder Tyrosinresten auf, und Proteinkinasen wurden deshalb nach ihrer Spezifität des Phosporylierungsortes, in Serin-/ Threonin-Kinasen und Tyrosin-Kinasen klassifiziert. Da Phosphorylierung ein sehr weit verbreiteter Prozess in Zellen ist, und da Zellphänotypen größtenteils von der Aktivität dieser Wege beeinflusst werden, wird zur Zeit angenommen, dass eine große Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (siehe Übersichtsartikel: Weinstein-Oppenheimer et al., Pharma. &. Therap. 88:229-279, 2000). Verschiedene Möglichkeiten zur Hemmung, Regulation und Modulation von Kinasen umfassen beispielsweise die Bereitstellung von Antikörpern, antisense-Ribozymen und Inhibitoren. In der Onkologieforschung sind bisher vor allem Tyrosinkinasen als vielversprechende Targets betrachtet worden. So sind zahlreiche synthetische kleine Moleküle als Tyrosinkinase-Inhibitoren zur Behandlung von Krebs in der klinischen Entwicklung z.B. Iressa^{®} oder Gleevec^{®}. Allerdings sind hier noch zahlreiche Probleme zu lösen, wie Nebenwirkungen, Dosierung, Resistenz des Tumors, Tumorspezifität und Patientenauswahl.

Bei den Serin/Threonin-Kinasen handelt es sich um eine Klasse von Enzymen, die die Übertragung des endständigen Phosphats des Adenosintriphosphats auf Serin- oder Threoninreste bei Proteinsubstraten katalysieren. Man nimmt an, dass den Serin/Threonin-Kinasen bei verschiedenen Zellfunktionen über die Substratphosphorylierung eine wesentliche Rolle bei der Signaltransduktion zukommt. Obwohl die genauen Mechanismen der Signaltransduktion noch unklar sind, wurde gezeigt, dass neben den Tyrosin-Kinasen auch die die Serin/Threonin-Kinasen wichtige Faktoren bei der Zellproliferation, der Karzinogenese und der Zelldifferenzierung darstellen.
Sie können daher an Krankheiten wie Krebs, Schuppenflechte und Hyperimmunreaktionen beteiligt sein.

Die vorliegende Erfindung betrifft nun Verbindungen der Formel AV, vorzugsweise als Regulatoren, Modulatoren oder Inhibitoren von Proteinkinasen, insbesondere des Typs Serin/Threonin-Kinase, zu denen unter anderem die Phosphoinositid-abhängige Kinase (PDK) gehören. Besondere Wirkung zeigen die erfindungsgemäßen Verbindungen bei der Inhibierung der Serin/Threonin-Kinase PDK1.

PDK1 phosphoryliert und aktiviert eine Untergruppe der AGC Proteinkinasen-Familie, umfassend PKB, SGK, S6K und PKC Isoformen. Diese Kinasen sind an dem PI3K Signalübertragungsweg beteiligt und kontrollieren grundlegende zelluläre Funktionen wie Überleben, Wachstum und Differenzierung. PDK1 ist somit ein bedeutender Regulator diverser metabolischer, proliferativer und Lebenserhaltungs-Effekte.

Durch Proteinkinasen wie PDK1 hervorgerufene Erkrankungen sind durch eine anomale Aktivität oder Hyperaktivität solcher Proteinkinasen gekennzeichnet. Anomale Aktivität betrifft entweder: (1) die Expression in Zellen, die gewöhnlich diese Proteinkinasen nicht exprimieren; (2) erhöhte Kinasen-Expression, die zu unerwünschter Zellproliferation, wie Krebs, führt; (3) erhöhte Kinasen-Aktivität, die zu unerwünschter Zellproliferation, wie Krebs, und/oder zu Hyperaktivität der entsprechenden Proteinkinasen führt. Hyperaktivität bezieht sich entweder auf eine Amplifikation des Gens, das eine bestimmte Proteinkinase codiert, oder die Erzeugung eines Aktivitäts-Spiegels, der mit einer Zellproliferationserkrankung korreliert werden kann (d.h. mit steigendem Kinase-Spiegel steigt die Schwere eines oder mehrerer Symptome der Zellproliferationserkrankung) die biologische Verfügbarkeit einer Proteinkinase kann auch durch das Vorhandensein oder Fehlen eines Satzes von Bindungsproteinen dieser Kinase beeinflusst werden.

Im Falle von PDK1 ist eine anomale Aktivität der Substrate PKB und S6K dieser Kinase in einer Vielzahl von Krebsarten beobachtet worden, die Punktmutationen des PTEN Gens aufweisen, was zu unkontollierter Proliferation und einer erhöhten Überlebensrate führt. Inhibitoren von PDK1 dürften sich daher als vorteilhaft bei der Behandlung von Krebszellen mit konstitutiv aktivierten AGC Kinasen erweisen.

Inhibitoren von PDK1 sind z.B. in der WO 04/048343 oder der WO 05/054238 offenbart.

Die wichtigsten Krebsarten, die unter Verwendung einer erfindungsgemäßen Verbindung behandelt werden können, umfassen Kolorektalkrebs, kleinzelligen Lungenkrebs, nicht-kleinzelligen Lungenkrebs, das multiple Myelom sowie das Nierenzellkarzinom und das Endometriumkarzinom, bersonders auch Krebsarten, in denen PTEN mutiert ist, u. a. Brustkrebs, Prostata-Krebs und Glioblastom.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Eine Reihe von Diazepinonen sind in der WO 04/076424 als Kinase-Inhibitoren beschrieben worden. Weitere Diazepinone sind offenbart in US 3872122 A, WO 81/00568 A sowie in den Publikationen von Palanki M.S.S. et al. (Med Chem Res 10:1 (2000) 19-29) und Dugosz E. (Pharmazie 50 (1995) 180-182).

Der Erfindung lag nunmehr die Aufgabe zugrunde, weitere Diazepinone mit vorteilhaften therapeutischen Eigenschaften aufzufinden, die zur Herstellung von Arzneimitteln verwendet werden können.

### Beschreibung der Erfindung

Es wurde gefunden, dass die Verbindungen der Formel AV und ihre Salze bei guter Verträglichkeit sehr wertvolle pharmakologische Eigenschaften besitzen. Insbesondere wurde gefunden, dass die erfindungsgegenständlichen Verbindungen der Formel AV überraschenderweise wirksame Kinase-Inhibitoren darstellen, wobei sie insbesondere eine Serin/Threonin-Kinase inhibierende Wirkung, und in besonderem Maße eine PDK1 inhibierende Wirkung zeigen.

Generell gilt, dass sämtliche Reste, die mehrfach auftreten, gleich oder verschieden sein können, d.h. unabhängig voneinander sind. Vor- und nachstehend haben die Reste bzw. Parameter die für die Formel AV angegebenen Bedeutungen, falls nicht ausdrücklich etwas anderes angegeben ist.

Dementsprechend sind Gegenstand der Erfindung insbesondere diejenigen Verbindungen der Formel AV, in denen mindestens einer der genannten Reste eine der nachstehend angegebenen bevorzugten Bedeutungen hat.

Hal bedeutet Fluor, Chlor, Brom oder Iod, insbesondere Fluor oder Chlor.

A bedeutet Alkyl, ist unverzweigt (linear), verzweigt oder cyclisch, und hat 1, 2, 3, 4, 5, 6, 7, 8, 9, 10, 11, 12, 13 oder 14 C-Atome.

So bedeutet A beispielsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1-oder 2-Ethylbutyl, 1-Ethyl-1-methylpropyl, 1-Ethyl-2-methylpropyl, 1,1,2-oder 1,2,2-Trimethylpropyl, lineares oder verzweigtes Heptyl, Octyl, Nonyl oder Decyl.

A bedeutet bevorzugt Alkyl mit 1, 2, 3, 4, 5 oder 6 C-Atomen, worin eine oder zwei CH₂-Gruppen durch O- oder S-Atome und/oder durch NH, NA, CONH, NHCO oder -CH=CH-Gruppen und/oder auch 1-7 H-Atome durch F und/oder Cl ersetzt sein können, wie z.B. Methyl, Ethyl, Propyl, Isopropyl, Butyl, Isobutyl, sek.-Butyl, tert.-Butyl, Pentyl, Hexyl, Trifluormethyl, Pentafluorethyl, 1,1-Difluormethyl, 1,1,1-Trifluorethyl, Methoxy, Ethoxy, n-Propoxy, Isopropoxy, n-Butoxy, Isobutoxy, sek.-Butoxy oder tert.-Butoxy, und worin eine oder zwei CH₃-Gruppen durch NH₂, NAH, NA₂ oder CN ersetzt sein können, wie bspw. N, N'-Dimethylaminoalkyl oder Cyanoalkyl.

Cycloalkyl bzw. cyclisches Alkyl bedeutet vorzugsweise Cyclopropyl, Cyclobutyl, Cylopentyl, Cyclohexyl oder Cycloheptyl.

Ar bedeutet z.B. unsubstituiertes Phenyl, Naphthyl oder Biphenyl, weiterhin vorzugsweise z.B. durch A, Fluor, Chlor, Brom, Iod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.

Ar bedeutet weiterhin Phenyl, o-, m- oder p-Tolyl, o-, m- oder p-Ethylphenyl, o-, m- oder p-Propylphenyl, o-, m- oder p-Isopropylphenyl, o-, m-oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m-oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m-oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-di-methylamino- oder 3-Nitro-4-N,N-dimethylaminophenyl, 2,3-Diamino-phenyl, 2,3,4-, 2,3,5-, 2,3,6-, 2,4,6- oder 3,4,5-Trichlorphenyl, 2,4,6-Tri-methoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet weiterhin unsubstituiertes oder ein-, zwei- oder dreifach z.B. durch Carbonylsauerstoff, F, Cl, Br, Methyl, Ethyl, Propyl, Phenyl, Benzyl,-CH₂-Cyclohexyl, Hydroxy, Methoxy, Ethoxy, Amino, Methylamino, Dimethylamino, Nitro, Cyan, Carboxy, Methoxycarbonyl, Aminocarbonyl, Methylaminocarbonyl, Dimethylaminocarbonyl, Acetamino, Ureido, Methylsulfonylamino, Formyl, Acetyl, Aminosulfonyl und/oder Methylsulfonyl substituiertes 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 2-, 3-, 5-, oder 6-Pyrazin-1- oder 4-yl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4-oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, 1-, 2-, 3-, 4-, 5-, 6- oder 7-Indolyl, 2-, 3-, 4- oder 5-Isoindolyl, 2-, 6, -oder 8-Purinyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 1-, 3-, 4-, 5-, 6-, 7- oder 8-Isochinolinyl, 3-, 4-, 5-, 6-, 7- oder 8-Chinolinyl, 2-, 4-, 5-, 6-, 7-oder 8-Chinazolinyl, 5- oder 6-Chinoxalinyl, 4-, 5-, oder 6-Phthalazinyl, 2-, 3-, 5-, 6-, 7- oder 8-2H-Benzo[1,4]oxazinyl, weiter bevorzugt 1,3-Benzo-dioxol-5-yl, 1,4-Benzodioxan-6-yl, 2,1,3-Benzothiadiazol-4-oder-5-yl oder 2,1,3-Benzoxadiazol-5-yl.

Die heterocyclischen Reste können auch teilweise oder vollständig hydriert sein und bedeuten z.B. auch 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 2-, 3-, 5- oder 6-Piperidin-1 oder 4-yl, 2-, 3- oder 4-Morpholinyl, Tetrahydro-2-,-3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2-oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)-phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Die Bezeichnung "substituiert" bezieht sich vorzugsweise auf die Substitution mit den obengenannten Substituenten, wobei mehrere unterschiedliche Substitutionsgrade möglich sind, falls nicht anders angegeben.

Erfindungsgemäß sind auch alle physiologisch unbedenklichen Salze, Derivate, Solvate und Stereoisomere dieser Verbindungen, einschließlich deren Mischungen in allen Verhältnissen.

Die Verbindungen der Formel AV können ein oder mehrere chirale Zentren aufweisen. Sie können dementsprechend in verschiedenen enantiomeren Formen auftreten und in racemischer oder in optisch aktiver Form vorliegen. Gegenstand der Erfindung sind deshalb auch die optisch aktiven Formen (Stereoisomeren), die Enantiomeren, die Racemate, die Diastereomeren sowie Hydrate und Solvate dieser Verbindungen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktive Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wässrige oder alkoholische Lösungsmittelgemische wie z.B. Hexan/Isopropanol/ Acetonitril z.B. im Verhältnis 82:15:3.
Eine elegante Methode zur Spaltung von Racematen mit Estergruppen (z.B. Acetylester) stellt die Verwendung von Enzymen, insbesondere Esterasen, dar.

Nachstehend wird die Bezeichnung R¹ stellvertretend für einen der Reste R^{1'}, R^{1"}, R^{1"'} oder R^{1""} und die Bezeichnung R² stellvertretend für einen der Reste R^{2'} oder R^{2"} verwendet.

Weiter bevorzugte Untergruppen von Verbindungen der Formel AV können durch die folgenden Teilformeln Aa bis Ah ausgedrückt werden, die der Formel AV entsprechen, worin jedoch
bei der Teilformel Aa
- X: CR¹ oder CHR¹ bedeutet
und alle übrigen Reste die für die Formel AV angegebene Bedeutung haben,
bei der Teilformel Ab
- einer der Reste X: N oder NR¹,
- die anderen drei Reste X: CR¹ oder CHR¹ bedeuten
und alle übrigen Reste die für die Formel AV angegebene Bedeutung haben,
bei der Teilformel Ac
- R^{5'}: Methyl bedeutet
und alle übrigen Reste die für die Formel AV angegebene Bedeutung haben,
bei der Teilformel Ad
- R³: H bedeutet
und alle übrigen Reste die für die Formel AV angegebene Bedeutung haben,
bei der Teilformel Ae
- R^{2'}, R^{2"}: H bedeuten
und alle übrigen Reste die für die Formel AV angegebene Bedeutung haben,
bei der Teilformel Af
- Y: NR⁴,
- R⁴: H oder Methyl bedeutet
und alle übrigen Reste die für die Formel AV angegebene Bedeutung haben,
bei der Teilformel Ag
- R^{1'}, R^{1"}: H,
- R^{1'"}: H, Hal oder Methyl,
- R^{1""}: H, Hal, Methyl, Ethyl, n-Propyl, 2-Propyl, Butyl, Isobutyl, sek.-Butyl tert.-Butyl, Methoxy, CHal₃, CF₃, OH, OCH₂CH₂OH, SCH₂CH₃, NHCH₃, N(CH₃)₂, CN, COOH, COOCH₃, SO₂OH, OCHal₃, OCF₃, NHCOA, NHCOAr, NHCON(R⁶)₂, NHCONHA, NHCONHAr ist, wobei A und R⁶ H, Cyclopentyl, Cyclohexyl, n-Propyl, 2-Propyl, Ethyl, sek.-Butyl oder tert.-Butyl sind und Ar Thiophen-2 oder 3-yl, 3,5-Demimethyl-isoxazol-4-yl ist,
und zwei Reste R6 gemeinsam mit dem Amid-Stickstoffatom
einen Tetrahydropyrrol-Ring bilden können,
und alle übrigen Reste die für die Formel AV angegebene Bedeutung haben,
bei der Teilformel Ah
- einer der Reste X: N oder NR¹,
- die anderen drei Reste X: CR¹ oder CHR¹,

- Y: NR⁴,
- R⁴: H oder Methyl,
- R^{5'}: Methyl,
- R^{2'}, R^{2"} R³: H,
- R^{1'}, R^{1"}: H,
- R^{1"'}: H, Hal oder Methyl,
- R^{1""}: H, Hal, Methyl, Ethyl, n-Propyl, 2-Propyl, Butyl, Isobutyl, sek.-Butyl tert.-Butyl, Methoxy, CHal₃, CF₃, OH, OCH₂CH₂OH, SCH₂CH₃, NHCH₃, N(CH₃)₂, CN, COOH, COOCH₃, SO₂OH, OCHal₃, OCF₃, NHCOA, NHCOAr, NHCON(R⁶)₂, NHCONHA, NHCONHAr ist, wobei A und R⁶ H, Cyclopentyl, Cyclohexyl, n-Propyl, 2-Propyl, Ethyl, sek.-Butyl oder tert.-Butyl sind und Ar Thiophen-2 oder 3-yl, 3,5-Demimethyl-isoxazol-4-yl ist, und zwei Reste R6 gemeinsam mit dem Amid-Stickstoffatom einen Tetrahydropyrrol-Ring bilden können,
und alle übrigen Reste die für die Formel AV angegebene Bedeutung haben
sowie ihre pharmazeutisch unbedenklichen Salze, Derivate, Solvate und Stereoisomere, einschließlich deren Mischungen in allen Verhältnissen.

Besonders bevorzugt sind Verbindungen, ausgewählt aus den in der Tabelle 1 aufgeführten Verbindungen sowie ihre pharmazeutisch unbedenklichen Salze, Derivate, Solvate und Stereoisomere, einschließlich deren Mischungen in allen Verhältnissen.

**Tabelle 1**

| | | **IC50 PDK1 (µM)** | **HPLC (min) (1)** | **HPLC-MS (min m/z)** | **Produkt** |
|---|---|---|---|---|---|
| 1 | 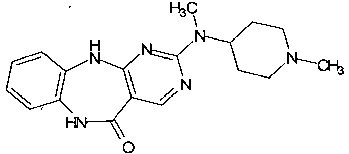 | 2,3 | 1,95 | 0,420 339,2 | orangebrauner Feststoff |
| 2 | 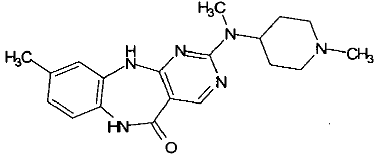 | 2,4 | 1,92 | 1,273 353,2 | orangebrauner Feststoff |
| 3 | 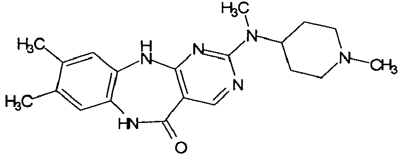 | 1,0 | 2,06 | 1,387 367,2 | orangebrauner Feststoff |
| 4 | 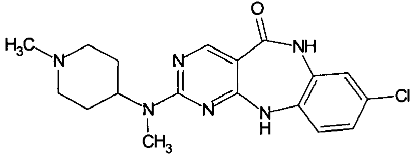 | 1,4 | 2,08 | 0,678 373,0 | orangebrauner Feststoff |
| 5 | 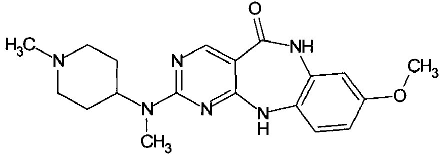 | 0,8 | 2,01 | 1,229 369,2 | orange-brauner Feststoff |
| 6 | 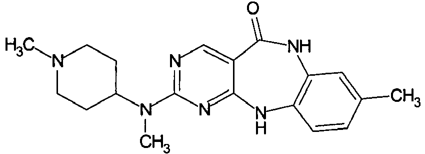 | 1,1 | 2,02 | 1,275 353,2 | orangebrauner Feststoff |
| 7 | 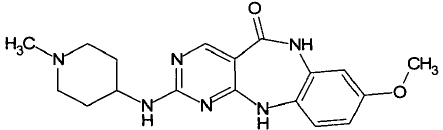 | 3,1 | 2,01 | 0,458 355,2 | orangebrauner Feststoff |
| 8 | 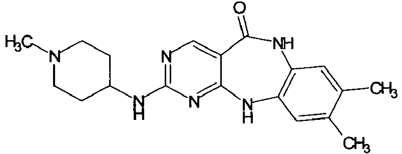 | 2,5 | 1,96 | 1,338 353,2 | orangebrauner Feststoff |
| 9 | 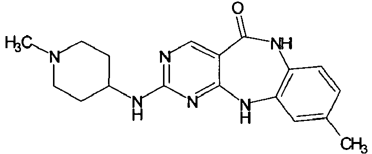 | 4,8 | 1,96 | 1,253 339,2 | orangebrauner Feststoff |
| 10 | 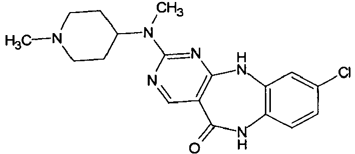 | 1,7 | 2,04 | 1,380 373,2 | orangebrauner Feststoff |
| 11 | 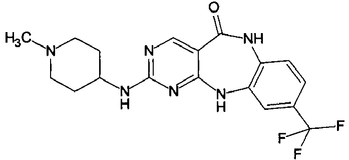 | 3,9 | 2,07 | 1,372 393,1 | orangebrauner Feststoff |
| 12 | 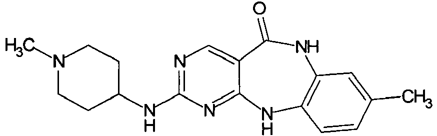 | 0,9 | 1,93 | 1,264 339,2 | orangebrauner Feststoff |
| 13 | 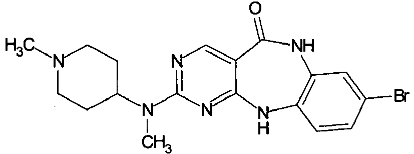 | 1,7 | 2,05 | 1,427 417,0 | orangebrauner Feststoff |
| 14 | 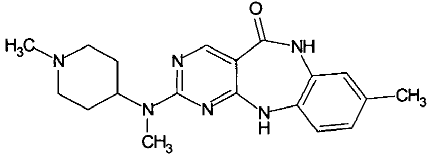 | 0,4 | 3,25 | 1,320 352,2 | gelbgrüner Feststoff |
| 15 | 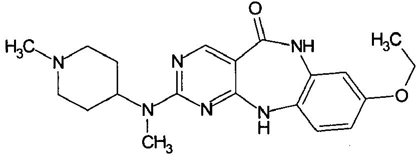 | 0,9 | 3,21 | 1,350 383,2 | gelber Feststoff |
| 16 | 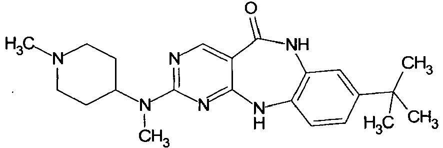 | 0,4 | 4,19 | 1,580 395,2 | gelber Feststoff |
| 17 | 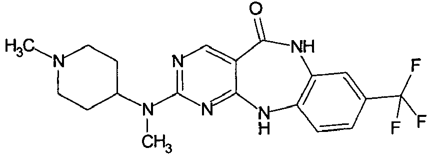 | 1,6 | 3,87 | 1,510 407,2 | gelber Feststoff |
| 18 | 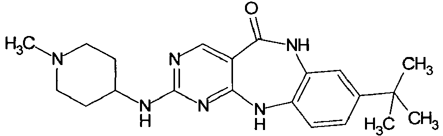 | 0,7 | 4,08 | 1,550 381,2 | gelber Feststoff |
| 19 | 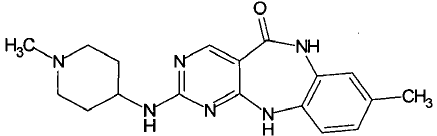 | 0,6 | 3,17 | 1,310 339,2 | gelber Feststoff |
| 20 | 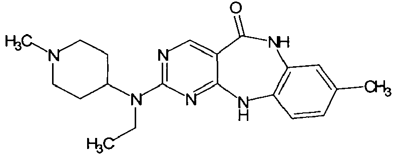 | 2,8 | 1,95 | 1,308 367,2 | orangebrauner Feststoff |
| 21 | 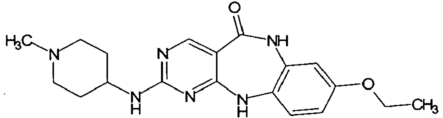 | 1,0 | 3,16 | 1,330 369,2 | gelber Feststoff |
| 22 | 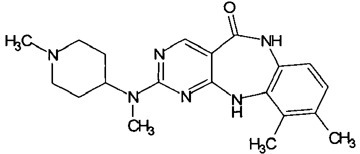 | 2,6 | 3,34 | 1,410 367,2 | gelber Feststoff |
| 23 | 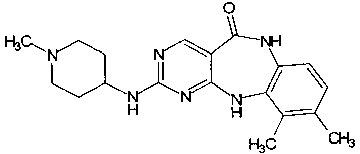 | 3,6 | 3,15 | 1,350 353,2 | gelber Feststoff |
| 24 | 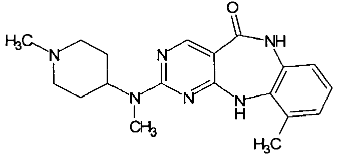 | | 3,23 | 1,320 353,2 | gelber Feststoff |
| 25 | 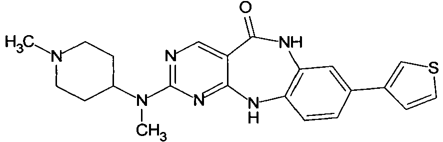 | 1,0 | 2,17 | 1,569 421,2 | orangener Feststoff |
| 26 | 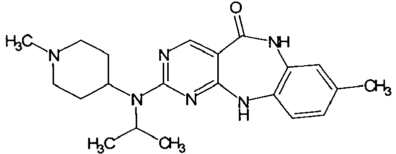 | 6,2 | 1,99 | 1,346 381,2 | orangener Feststoff |
| 27 | 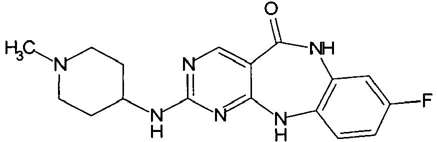 | | 2,96 | 1,260 343,2 | gelber Feststoff |
| 28 | 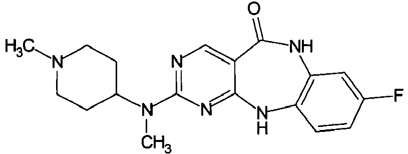 | | 3,07 | 1,270 357,2 | gelber Feststoff |
| 29 | 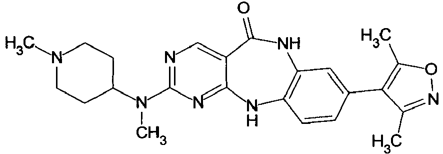 | 3,2 | 2,05 | 1,399 434,2 | orangener Feststoff |
| 30 | 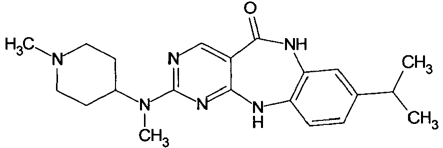 | | 3,6 | 1,480 381,2 | gelber Feststoff |
| 31 | 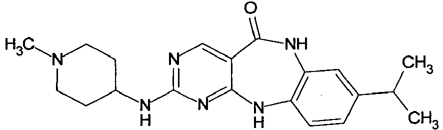 | | 3,55 | 1,500 367,2 | gelber Feststoff |
| | | **IC50 PDK1 (M)** | **HPLC (min) (2)** | **MS (min m/z)** | **Produkt** |
| 32 | 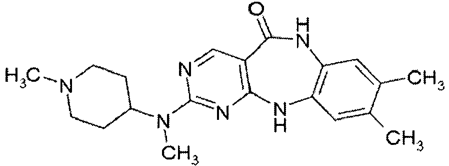 | 6,4E-07 | 3,14 | 366,5 | beiges Pulver |
| 33 | 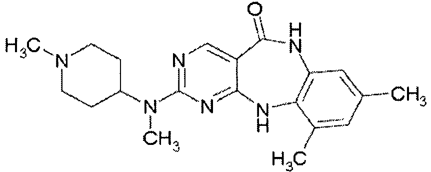 | 1,3E-06 | 3,35 | 366,5 | gelb, fest |
| 34 | 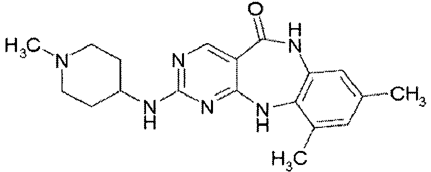 | 3,7E-06 | 3,17 | 352,4 | gelb,fest |
| 35 | 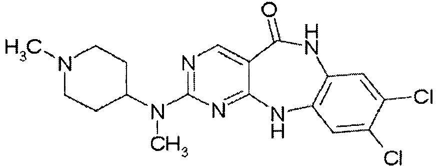 | 3,7E-06 | 3,79 | 407,3 | gelb, fest |
| 36 | 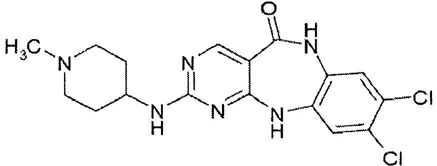 | 1,2E-06 | 3,61 | 393,3 | gelb, fest |
| 37 | 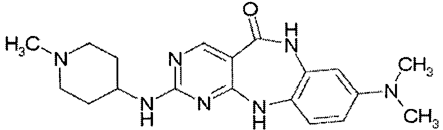 | 1,9E-07 | 2,53 | 367,5 | gelb, flockig |
| 38 | 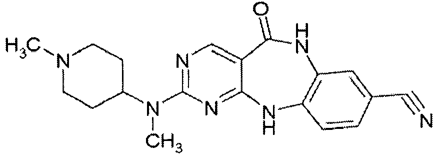 | 6,0E-07 | 3,2 | 363,4 | gelb, flockig |
| 39 | 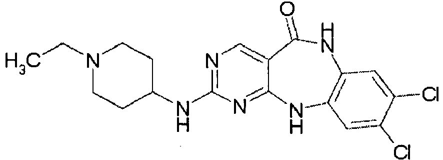 | 4,9E-06 | 3,74 | 407,3 | hellgelb, fest |
| 40 | 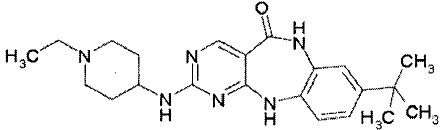 | 1,4E-06 | 4,19 | 394,5 | gelb, flockig |
| 41 | 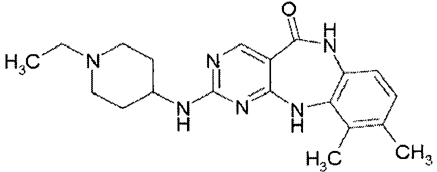 | 6,3E-06 | 3,17 | 366,5 | gelb, fest |
| 42 | 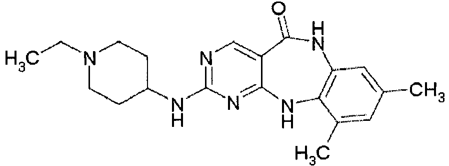 | 4,8E-06 | 3,21 | 366,5 | gelb, fest |
| 43 | 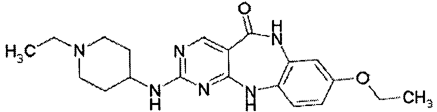 | 2,0E-06 | 3,13 | 382,5 | gelb, fest |
| 44 | 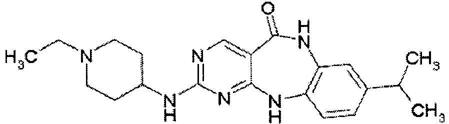 | 9,2E-07 | 3,56 | 380,5 | gelb, fest |
| 45 | 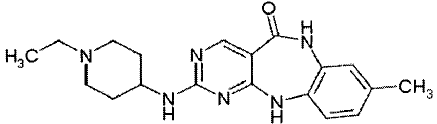 | 1,5E-06 | 3,33 | 352,4 | gelb, flockig |
| 46 | 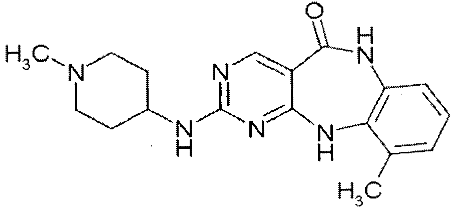 | 2,4E-06 | 3,07 | 338,4 | gelb, flockig |
| 47 | 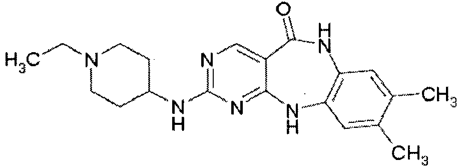 | 1,5E-06 | 3,6 | 366,5 | gelb, flockig |
| 48 | 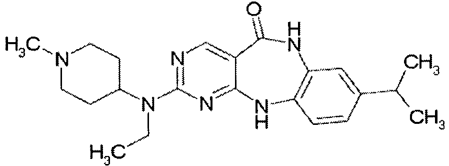 | 3,0E-06 | 3,66 | 394,5 | gelb, fest |
| 49 | 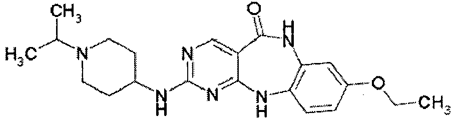 | 5,1E-06 | 3,34 | 396,5 | gelb, fest |
| 50 | 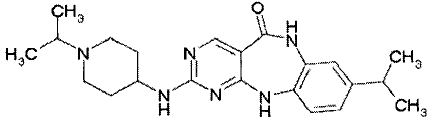 | 1,2E-06 | 3,8 | 394,5 | gelb, fest |
| 51 | 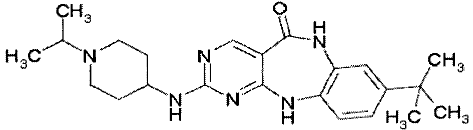 | 2,7E-06 | 4,2 | 408,5 | gelb, flockig |
| 52 | 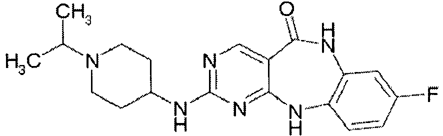 | 3,6E-06 | 3,04 / 3,11* | 370,4 | gelb, flockig |
| 53 | 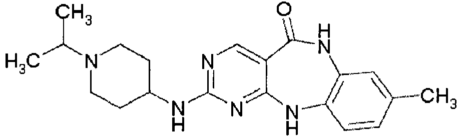 | 2,4E-06 | 3,36 | 366,5 | gelb, flockig |
| 54 | 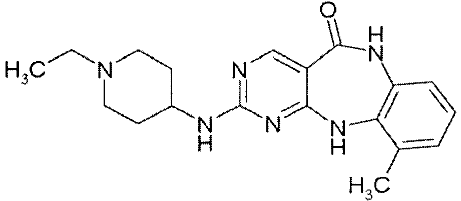 | 7,1E-06 | 3,17 | 352,4 | gelb, flockig |
| 55 | 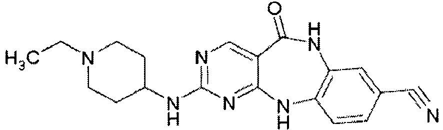 | 2,3E-06 | 2,68 / 2,77* | 363,4 | gelb, flockig |
| 56 | 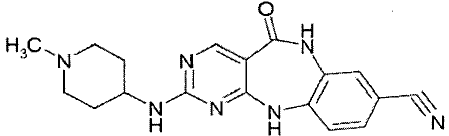 | 1,5E-06 | 2,59 / 2,69* | 349,4 | gelb, flockig |
| 57 | 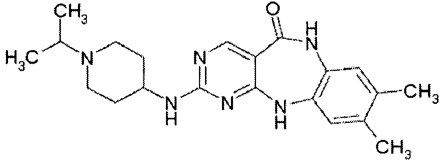 | 2,0E-06 | 3,48 | 380,5 | gelb, flockig |
| 58 | 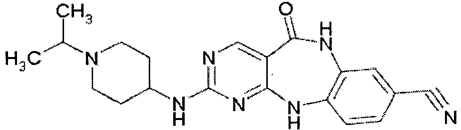 | 7,1E-06 | 2,82 / 2,90* | 377,5 | gelb, flockig |
| 59 | 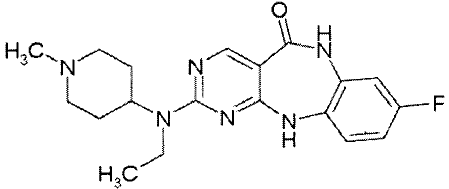 | 2,4E-06 | 2,74 / 2,83* | 370,4 | gelb, flockig |
| 60 | 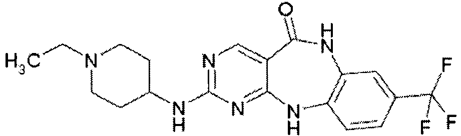 | 2,9E-06 | 3,41 | 406,4 | gelb, flockig |
| 61 | 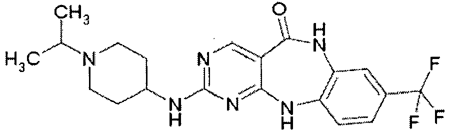 | 6,5E-06 | 3,61 | 420,4 | gelb, flockig |
| 62 | 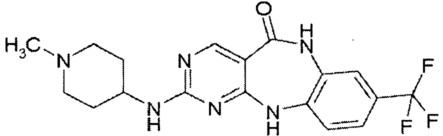 | 1,8E-06 | 3,49 | 392,4 | gelb, flockig |
| 63 | 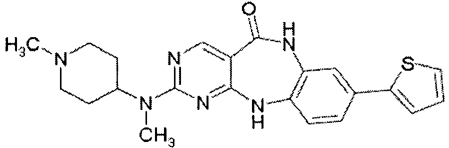 | 1,0E-06 | 3,91 | 420,5 | gelb, fest |
| 64 | 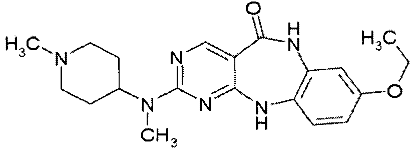 | 4,3E-07 | 3,11 | 382,5 | gelb, fest |
| 65 | 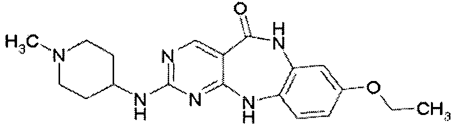 | 5,2E-07 | 3,1 | 368,4 | gelb, fest |
| 66 | 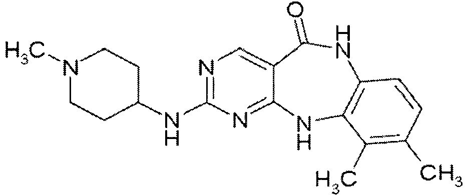 | 3,4E-06 | 3,19 / 3,28* | 352,4 | beiges Pulver |
| 67 | 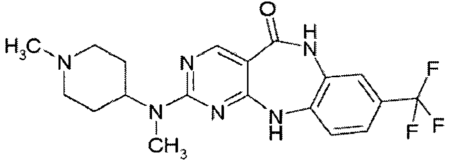 | 1,3E-06 | 3,79 | 406,4 | gelbes Pulver |
| 68 | 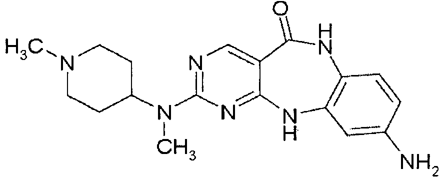 | 5,1E-07 | 2,16 | 353,4 | hellgelb, fest |
| 69 | 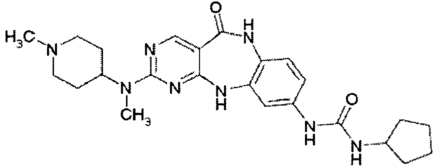 | 6,5E-07 | 3,55 | 464,6 | gelb, fest |
| 70 | 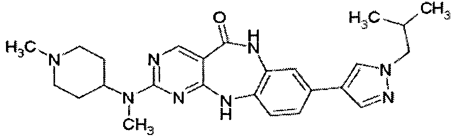 | 1,1E-07 | 3,68 | 460,6 | gelb, fest |
| 71 | 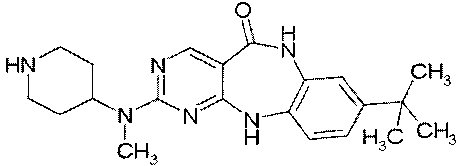 | 4,3E-07 | 4,07 | 380,5 | gelb, flockig |
| 72 | 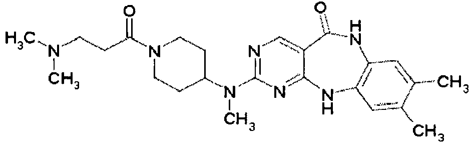 | 8,1E-06 | 3,56 / 3,60* | 451,6 | gelb, flockig |
| 73 | 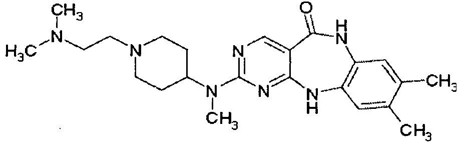 | 3,9E-06 | 3,26 / 3,32* | 423,6 | gelb, flockig |
| 74 | 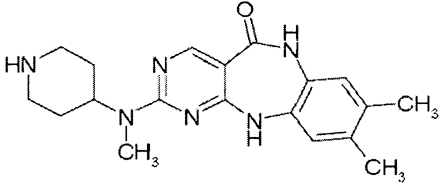 | 3,2E-07 | 3,34 / 3,38* | 352,4 | beiges Pulver |
| 75 | 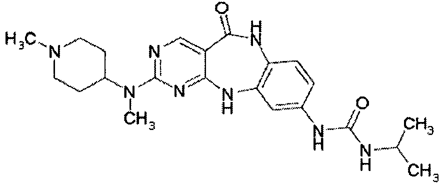 | 9,6E-07 | 3,17 | 438,5 | hellgelb, fest |
| 76 | 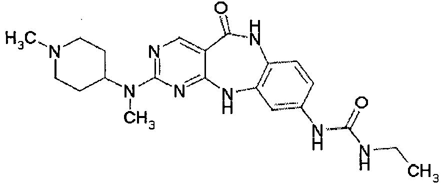 | 1,3E-06 | 2,99 | 424,5 | gelb, fest |
| 77 | 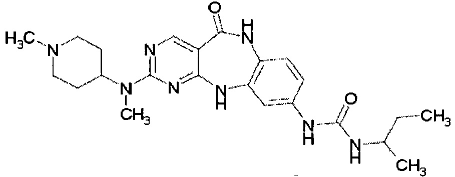 | 9,4E-07 | 3,41 | 452,6 | gelb, fest |
| 78 | 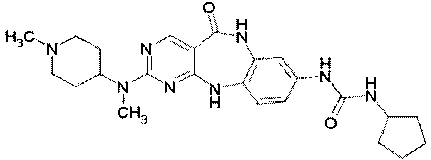 | 1,5E-07 | 3,57 | 464,6 | gelb, fest |
| 79 | 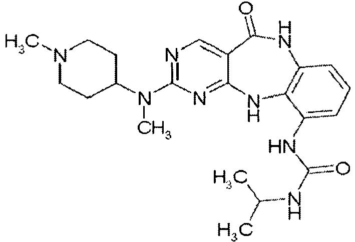 | 6,0E-06 | 2,88 | 438,5 | hellgelb, fest |
| 80 | 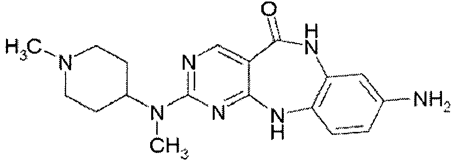 | 1,9E-07 | 2,16 | 353,4 | gelb, fest |
| 81 | 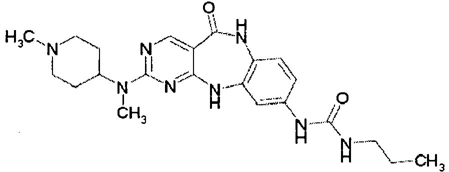 | 4,2E-07 | 3,23 | 438,5 | gelb, fest |
| 82 | 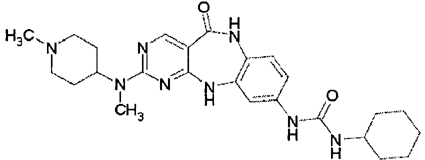 | 7,5E-07 | 3,84 | 478,6 | hellgelb, fest |
| 83 | 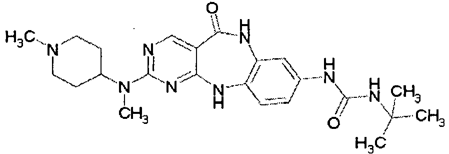 | 2,0E-07 | 3,52 | 452,6 | gelb, fest |
| 84 | 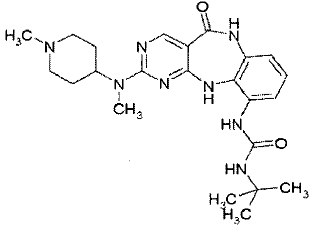 | 5,5E-06 | 3,25 | 452,6 | gelb, fest |
| 85 | 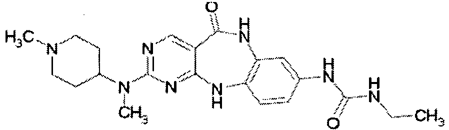 | 2,8E-07 | 2,99 | 424,5 | gelb, fest |
| 86 | 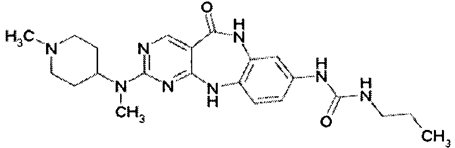 | 2,8E-07 | 3,23 | 438,5 | gelb, fest |
| 87 | 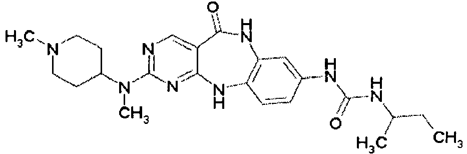 | 2,3E-07 | 3,57 | 452,6 | gelb, fest |
| 88 | 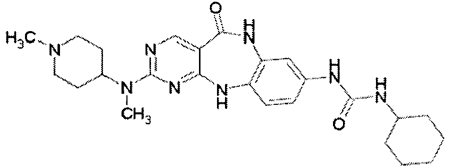 | 1,5E-07 | 3,84 | 478,6 | gelb, fest |
| 89 | 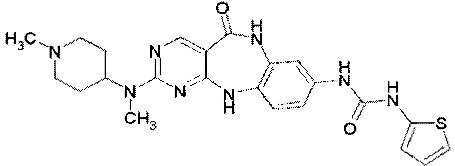 | 2,0E-07 | 3,63 | 478,6 | gelb, fest |
| 90 | 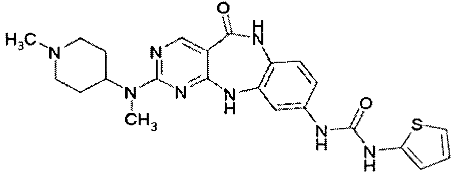 | 2,9E-07 | 3,63 | 478,6 | gelb, fest |
| 91 | 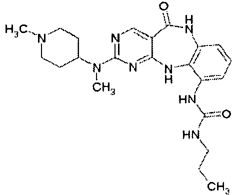 | | 2,91 | 438,5 | gelb, fest |
| 92 | 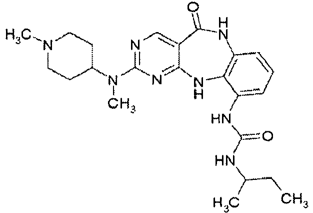 | 7,8E-06 | 3,17 | 452,6 | hellgelb, fest |
| 93 | 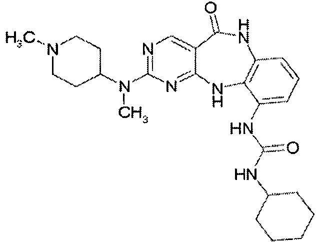 | 3,5E-06 | 3,57 | 478,6 | hellgelb, fest |
| 94 | 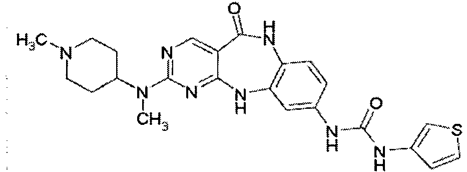 | 1,6E-07 | 3,65 | 478,6 | hellgelb, fest |
| 95 | 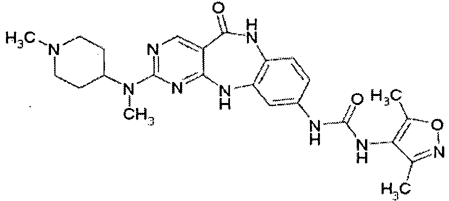 | 9,5E-07 | 3,15 | 491,6 | hellgelb, fest |
| 96 | 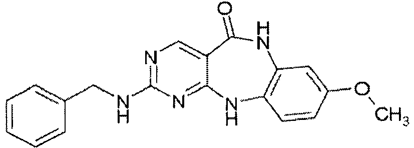 | | 4,49 | | beige, flockig |
| 97 | 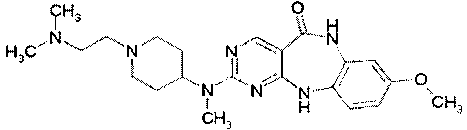 | | 3,96 | 425,5 | gelb, flockig |
| 98 | 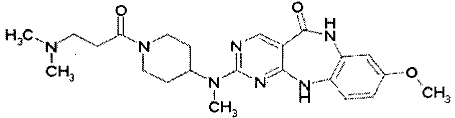 | | 3,13 / 3,20* | 453,5 | gelb, flockig |
| 99 | 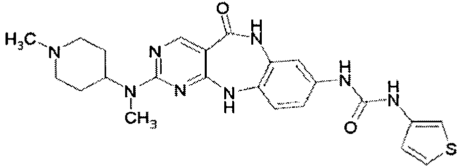 | 1,2E-07 | 3,65 | 478,6 | gelb, fest |
| 100 | 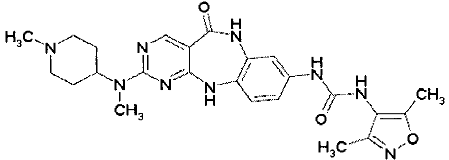 | 2,2E-07 | 3,12 | 491,6 | gelb, fest |
| 101 | 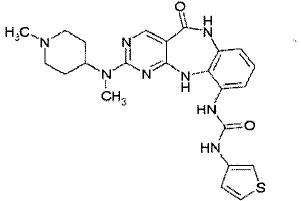 | 3,4E-07 | 3,2 | 478,6 | hellgelb, fest |
| 102 | 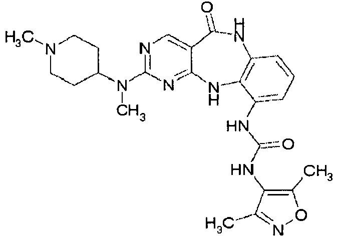 | 3,4E-06 | 2,91 | 491,6 | hellgelb, fest |
| 103 | 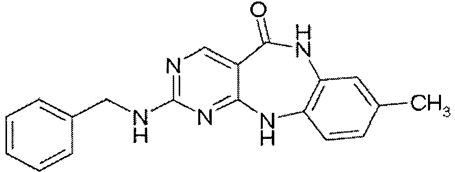 | | 4,62 | 331,4 | gelb, flockig |
| 104 | 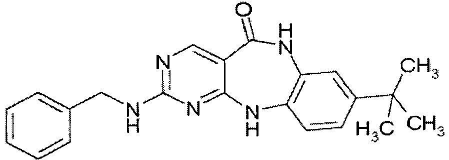 | | 5,54 | 373,5 | gelb, flockig |
| 105 | 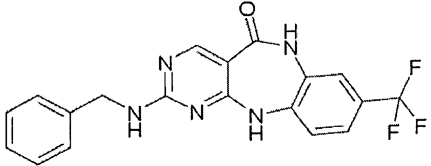 | | 5,18 | 385,3 | gelb, flockig |
| 106 | 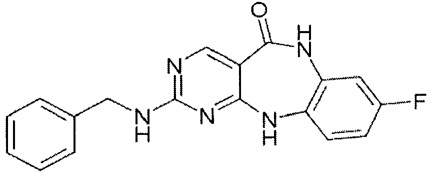 | | 4,44 | 335,3 | gelb, flockig |
| 107 | 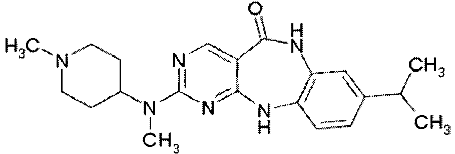 | | 3,67 | 380,5 | hellgelb, fest |
| 108 | 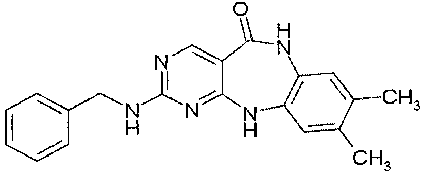 | | 2,96 | 345,4 | beiges Pulver |
| 109 | 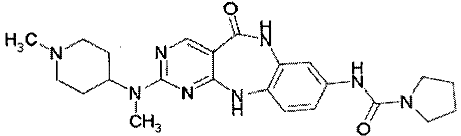 | | 3,23 | 450,5 | gelb, fest |
| 110 | 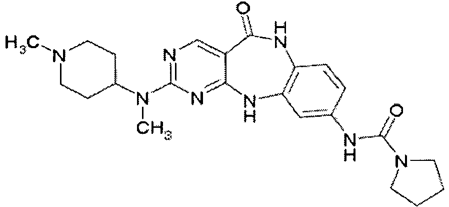 | | 3,23 | 450,5 | gelb, fest |
| 111 | 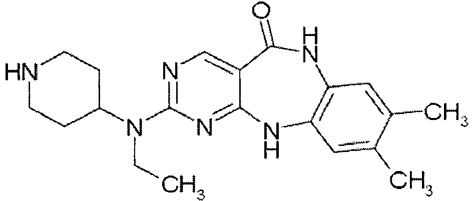 | | 3,51 | 366,5 | gelb, flockig |
| 112 | 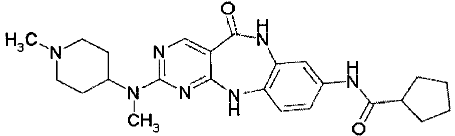 | | 3,6 | 449,6 | gelb, fest |
| 113 | 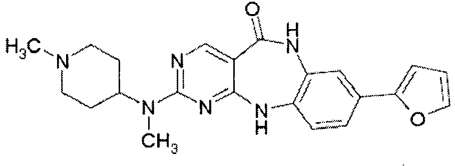 | | 3,73 | 404,5 | gelb, fest |
| 114 | 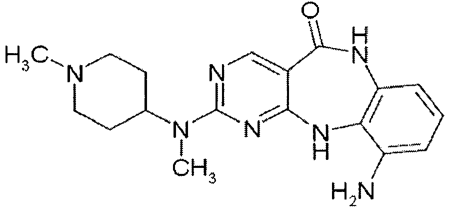 | | 2,61 | 353,4 | hellgelb, fest |
| 115 | 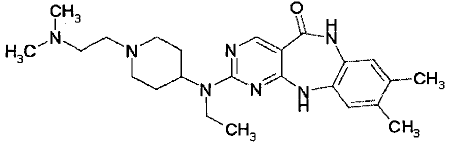 | | 3,4 | 437,6 | gelb, flockig |
| 116 | 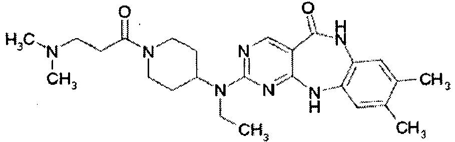 | | 3,75 | 465,6 | gelb, fest |
| 117 | 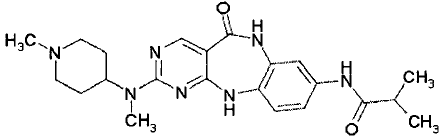 | | 3,17 | 423,5 | gelb, fest |
| 118 | 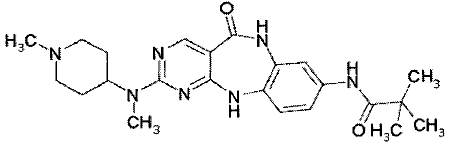 | | 3,41 | 437,5 | gelb, fest |
| 119 | 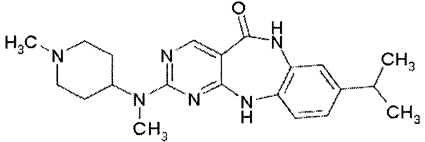 | | 3,7 | 380,5 | gelb, fest |
| 120 | 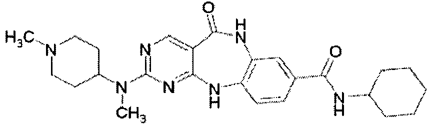 | | 3,6 | 463,6 | gelb, fest |
| 121 | 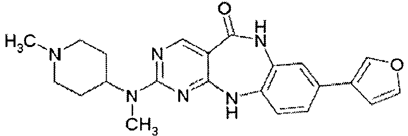 | | 3,57 | 404,5 | gelb, fest |
| 122 | 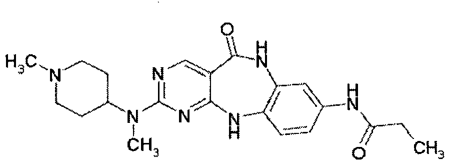 | | 2,99 | 409,5 | gelb, fest |
| 123 | 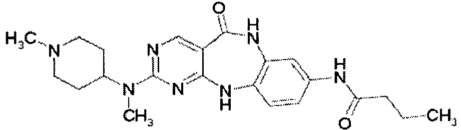 | | 3,2 | 423,5 | gelb, fest |
| 124 | 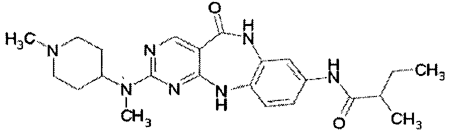 | | 3,39 | 437,5 | gelb, fest |

Unter pharmazeutisch oder physiologisch unbedenklichen Derivaten versteht man z.B. Salze der erfindungsgemäßen Verbindungen, als auch sogenannte Prodrug-Verbindungen. Solche Derivate sind in dem Fachmann bekannt. Eine Übersicht zu physiologisch verträglichen Derivaten liefert Burger's Medicinal Chemistry And Drug Discovery, 5th Edition, Vol 1: Principles and Practice. Unter Prodrug-Verbindungen versteht man mit z.B. Alkyl- oder Acylgruppen, Zuckern oder Oligopeptiden abgewandelte Verbindungen der Formel AV, die im Organismus rasch zu den wirksamen erfindungsgemäßen Verbindungen gespalten oder freigesetzt werden. Hierzu gehören auch bioabbaubare Polymerderivate der erfindungsgemäßen Verbindungen, wie dies z.B. in Int. J. Pharm. 115:61-67 (1995) beschrieben ist.

Als Säureadditionssalze kommen anorganische oder organische Salze aller physiologisch oder pharmakologisch unbedenklichen Säuren in Frage, beispielsweise Halogenide, insbesondere Hydrochloride oder Hydrobromide, Lactate, Sulfate, Citrate, Tartrate, Maleate, Fumarate, Oxalate, Acetate, Phosphate, Methylsulfonate oder p-Toluolsulfonate.

Unter Solvaten der Verbindungen der Formel AV werden Anlagerungen von inerten Lösungsmittelmolekülen an die Verbindungen der Formel AV verstanden, die sich aufgrund ihrer gegenseitigen Anziehungskraft ausbilden. Solvate sind beispielsweise Hydrate, wie Monohydrate oder Dihydrate oder Alkoholate, d.h. Additionsverbindungen mit Alkoholen wie beispielsweise mit Methanol oder Ethanol.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Gegenstand der Erfindung sind auch Mischungen der erfindungsgemäßen Verbindungen der Formel AV, z.B. Gemische zweier Diastereomere z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Weiterhin betrifft die vorliegende Erfindung ein Verfahren zur Herstellung von Verbindungen der Formel AV sowie ihrer physiologisch unbedenklichen Salze, Derivate, Solvate und Stereoisomere, dadurch gekennzeichnet, dass man in einem ersten Schritt eine Verbindung der Formel VIII 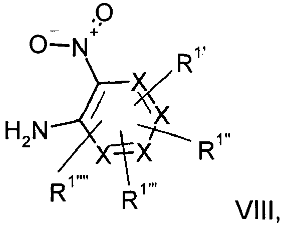 worin sämtliche Reste die zuvor angegebene Bedeutung haben mit einer Verbindung der Formel VII 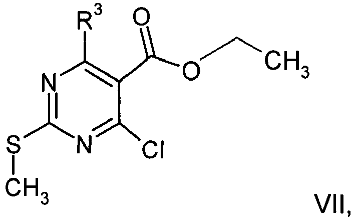 worin sämtliche Reste die zuvor angegebene Bedeutungen haben, zu einer Verbindung der Formel VI umsetzt 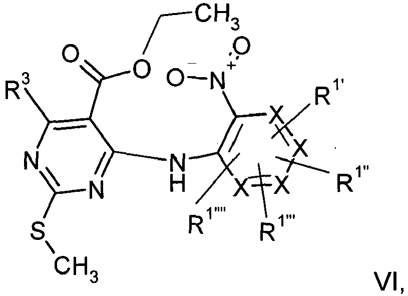 die zu einer Verbindung der Formel V 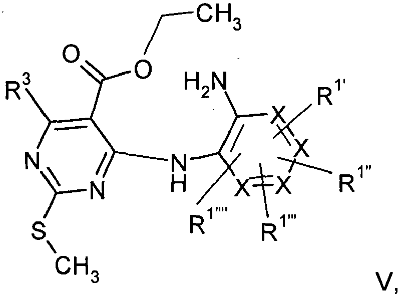 reduziert wird, welche dann im nächsten Schritt zu einer Verbindung der Formel IV 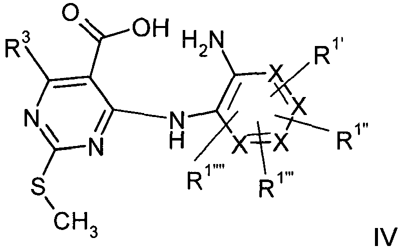 verseift wird, die weiter zu einem Diazepinon der Formel III 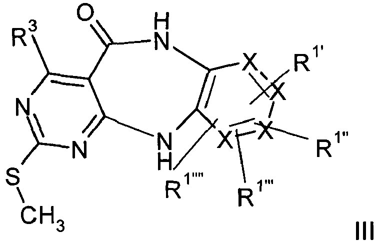 cyclisiert wird, das nach Erhöhung der Reaktivität des Thioethers, z.B. durch Oxidation zu einem Sulfon, mit einer Verbindung der Formel II 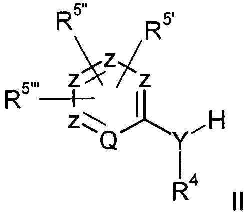 substituiert wird, wobei eine Verbindung der Formel Ib 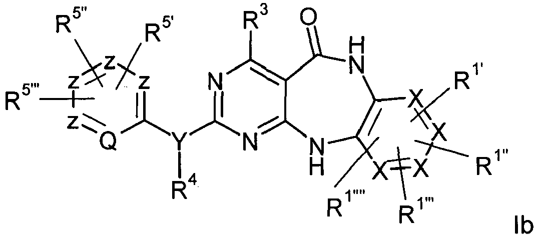 erhalten wird, die schließlich, falls die Reste R^{2'}, R^{2"} eine andere Bedeutung als H haben, zu einer Verbindung der Formel AV umgesetzt wird

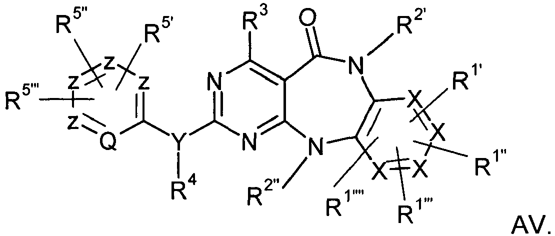

Die Verbindungen der Formel VIII, VII und II sind in der Regel bekannt. Sind sie neu, so können sie nach an sich bekannten Methoden hergestellt werden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind.

Die Verbindungen der Formel AV und auch die Ausgangsstoffe zu ihrer Herstellung werden nach an sich bekannten Methoden hergestellt, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Diazepinone der Formel AV können vorzugsweise erhalten werden, indem man wie folgt vorgeht:
a) Eine Verbindung der Formel VIII wird mit einer Verbindung der Formel VIII versetzt, wobei wahlweise ohne Lösungsmittel oder in einem inerten Lösungsmittel gearbeitet wird, und das Reaktionsgemisch bei erhöhter Temperatur gerührt. Nach Beendigung der Reaktion wird die Verbindung der Formel VI aus dem Reaktionsgemisch chromatographisch oder nach Fällen als Feststoff, vorzugsweise kristallin, isoliert.
b) Das Produkt aus (a) wird mittels eines geeigneten Katalysators bei Raumtemperatur und Normaldruck zu einer Verbindung der Formel V hydriert.
c) Das Reaktionsprodukt aus Schritt (b) wird bei erhöhter Temperatur verseift und die dabei erhaltene Verbindung der Formel IV aufgereinigt und aus dem Reaktionsgemsich abgetrennt.
d) Das Produkt aus (c) wird nun unter Zuhilfenahme geeigneter Kupplungsreagenzien zu einer Verbindung der Formel III cyclisiert und gereinigt.
e) Anschließend wird der im Schritt (d) erhaltene Thioether zur Erhöhung der Reaktivität mit einem Agens wie meta-Chlorperbenzoesäure in THF, Dichlormethan, Methyljodid in Acetonitril oder Chlor in THF behandelt.
f) Zum Schluß wird die so vorbehandelte Verbindung der Formel III mit einer Verbindung der Formel II nukleophil substituiert und dabei eine Verbindung der Formel AV erhalten, die - bspw. chromatographisch - aufgereinigt wird.

Die zuvor beschriebenen Umsetzungen erfolgen in der Regel in einem inerten Lösungsmittel. Als inerte Lösungsmittel für die zuvor beschriebenen Umsetzungen eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Ether wie Diethylether, Diisopropylether, Tetrahydrofuran (THF) oder Dioxan; Glykolether wie Ethylenglykolmonomethyl- oder monoethylether (Methylglykol oder Ethylglykol), Ethylenglykoldimethylether (Diglyme); Ketone wie Aceton oder Butanon; Amide wie Acetamid, N-Methyl-pyrrolidon (NMP), Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Bevorzugt sind Sulfoxide wie Dimethylsulfoxid (DMSO).

Die Menge des Lösungsmittels ist nicht kritisch, vorzugsweise können 5 g bis 500 g Lösungsmittel je g des zu bildenden Produkts zugesetzt werden.

In der Regel wird bei einem Druck von 1 bis 200 bar gearbeitet, bevorzugt jedoch bei Normaldruck.

Die Reaktionstemperatur für die zuvor beschriebenen Umsetzungen liegt je nach den angewendeten Bedingungen zwischen etwa -10 und 200 °C, normalerweise zwischen -5 und 100 °C, bevorzugt zwischen 0 und 80 °C.

Die Reaktionszeit liegt je nach den angewendeten Bedingungen zwischen einigen Minuten und mehreren Tagen, vorzugsweise im Bereich von mehreren Stunden.

Die Reaktion kann auch in heterogener Phase ausgeführt werden, wobei vorzugsweise eine wässrige Phase und eine Benzol- oder Toluol-Phase, eine feste und eine Dichlormethan- oder Chloroform-Phase und eine THF-Phase verwendet werden. Hier kommt ein Phasentransfer-Katalysator zum Einsatz, wie beispielsweise Tetrabutylammoniumiodid und gegebenenfalls ein Acylierungskatalysator, wie beispielsweise Dimethylaminopyridin.

Eine erhaltene Base der Formel AV kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-mono- und -disulfonsäuren, Laurylschwefelsäure.

Die freien Basen der Formel AV können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen.

Verbindungen der Formel AV können ferner erhalten werden, indem man sie aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel AV entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel AV entsprechen, jedoch anstelle einer Gruppe -COOH eine Gruppe -COOR" tragen, worin R" eine Hydroxyschutzgruppe bedeutet.
Bevorzugte Ausgangsstoffe sind auch die Oxadiazolderivate, die in die entsprechenden Amidinoverbindungen überführt werden können.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Tolyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Ferner kann man freie Aminogruppen in üblicher Weise mit einem Säurechlorid oder -anhydrid acylieren oder mit einem unsubstituierten oder substituierten Alkylhalogenid alkylieren, oder mit CH3-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30 °C.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkyl- oder Silylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel AV aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50 °C, vorzugsweise arbeitet man zwischen 15 und 30 °C (Raumtemperatur, RT).

Die Gruppen BOC, OBut und Mtr können z. B. bevorzugt mit TFA in Dichlormethan oder mit etwa 3 bis 5n HCl in Dioxan bei 15-30 °C abgespalten werden, die FMOC-Gruppe mit einer etwa 5- bis 50 %igen Lösung von Dimethylamin, Diethylamin oder Piperidin in DMF bei 15-30 °C.

Hydrogenolytisch entfernbare Schutzgruppen (z. B. CBZ, Benzyl oder die Freisetzung der Amidinogruppe aus ihrem Oxadiazolderivat) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100 °C und Drucken zwischen etwa 1 und 200 bar, bevorzugt bei 20-30 °C und 1-10 bar durchgeführt. Eine Hydrogenolyse der CBZ-Gruppe gelingt z.B. gut an 5 bis 10 %igem Pd/C in Methanol oder mit Ammomiumformiat (anstelle von Wasserstoff) an Pd/C in Methanol/DMF bei 20-30 °C.

Ester können z.B. mit Essigsäure oder mit NaOH oder KOH in Wasser, Wasser-THF oder Wasser-Dioxan bei Temperaturen zwischen 0 und 100 °C verseift werden.

Weitere Methoden zur Entfernung von Schutzgruppen ist beispielsweise in Theodora W. Green, Peter G. M. Wuts: Protective Groups in Organic Synthesis, 3rd Edition John Wiley & Sons (1999) beschrieben.

Erfindungsgemäße Verbindungen der Formel AV können aufgrund ihrer Molekülstruktur chiral sein und dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische, biochemische oder physikalische Maßnahmen, aufgetrennt oder bereits als solche bei der Synthese eingesetzt werden.

Durch übliche Aufarbeitungsschritte wie z.B. Wasserzugabe zum Reaktionsgemisch und Extraktion können die Verbindungen der Formel AV nach Entfernung des Lösungsmittels erhalten werden. Es kann vorteilhaft sein, zur weiteren Reinigung des Produktes eine Destillation oder Kristallisation anzuschließen.

Ein weiterer Gegenstand der Erfindung sind Arzneimittel, enthaltend wenigstens eine erfindungsgemäße Verbindung und/oder ihre physiologisch unbedenklichen Salze, Derivate, Solvate und Stereoisomere, einschließlich deren Mischungen in allen Verhältnissen.
Weiterhin kann eine erfindungsgemäße pharmazeutische Zubereitung, weitere Träger- und/oder Hilfsstoffe sowie gegebenenfalls einen oder mehrere weitere Arzneimittelwirkstoffe enthalten.

Gegenstand der Erfindung ist ferner ein Verfahren zur Herstellung eines Arzneimittels, dadurch gekennzeichnet, dass man eine erfindungsgemäße Verbindung und/oder eines ihrer physiologisch unbedenklichen Salze, Derivate, Solvate und Stereoisomere, einschließlich deren Mischungen in allen Verhältnissen zusammen mit einem festen, flüssigen oder halbflüssigen Träger- oder Hilfsstoff in eine geeignete Dosierungsform bringt.

Gegenstand der Erfindung ist auch ein Set (Kit) bestehend aus getrennten Packungen von
a) einer wirksamen Menge einer erfindungsgemäßen Verbindung und/oder ihrer physiologisch unbedenklichen Salze, Derivate, Solvate und Stereoisomere, einschließlich deren Mischungen in allen Verhältnissen und
b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Derivate, Solvate und Stereoisomere, einschließlich deren Mischungen in allen Verhältnissen, und einer wirksamen Menge eines weiteren Arzneimittelwirkstoffs gelöst oder in lyophylisierter Form vorliegt.

Arzneimittel können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Geschlecht, Gewicht und Zustand des Patienten. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche Arzneimitel mit einem der im pharmazeutischen Fachgebiet allgemein bekannten Verfahren herstellen.

Arzneimittel lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste Arzneimittel können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern. Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspart-amidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste Arzneimittel können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6):318 (1986) allgemein beschrieben.

An die topische Verabreichung angepasste Arzneimittel können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepassten Arzneimittel gehören Augentropfen, wobei der Wirkstoff in einem geeigneten Träger, insbesondere einem wässrigen Lösungsmittel, gelöst oder suspendiert ist.

An die topische Applikation im Mund angepasste Arzneimittel umfassen Lutschtabletten, Pastillen und Mundspülmittel.

An die rektale Verabreichung angepasste Arzneimittel können in Form von Zäpfchen oder Einläufen dargereicht werden.

An die nasale Verabreichung angepasste Arzneimittel in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste Arzneimittel umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepasste Arzneimittel können als Pessare, Tampons, Cremes, Gele, Pasten, Schäume oder Sprayformulierungen dargereicht werden.

Zu den an die parenterale Verabreichung angepassten Arzneimittel gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, dass die erfindungsgemäßen Arzneimittel neben den obigen besonders erwähnten Bestandteilen andere im Fachgebiet übliche Mittel mit Bezug auf die jeweilige Art der pharmazeutischen Formulierung enthalten können; so können beispielsweise für die orale Verabreichung geeignete Arzneimittel Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Empfängers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer Verbindung der Formel AV für die Behandlung der erfindungsgemäßen Erkrankungen im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden.

Die erfindungsgemäßen Verbindungen zeigen eine vorteilhafte biologische Aktivität, die in Enzym-Assays leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Gegenstand der vorliegenden Erfindung sind erfindungsgemäße Verbindungen als Effektoren, bevorzugt als Inhibitoren der hier beschriebenen Signalwege. Besonders bevorzugter Gegenstand der Erfindung sind deshalb erfindungsgemäße Verbindungen als Aktivatoren und Inhibitoren von Proteinkinasen, bevorzugt als Inhibitoren von Serin/Threonin-Kinasen, insbesondere der Phosphoinositid-abhängigen Kinase (PDK). Hierbei zeigen die erfindungsgemäßen Verbindungen eine besondere Wirkung bei der Inhibierung der Serin/Threonin-Kinase PDK1.

Wie vorstehend besprochen, sind die durch die erfindungsgemäßen Verbindungen beeinflussten Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind.

Ein weiterer Gegenstand der vorliegenden Erfindung ist deshalb die Verwendung von erfindungsgemäßen Verbindungen und/oder ihrer physiologisch unbedenklichen Salze, Derivate, Solvate und Stereoisomere, einschließlich deren Mischungen in allen Verhältnissen zur Herstellung eines Medikaments zur Behandlung und/oder Prophylaxe von Krankheiten, insbesondere solcher Krankheiten, die durch Proteinkinasen und/oder durch kinase-vermittelte Signaltransduktion verursacht, vermittelt und/oder propagiert werden. Bevorzugt sind hierbei Serin/Threonin-Kinasen, besonders bevorzugt ist PDK1.

Außerdem eignen sich die vorliegenden Verbindungen als pharmazeutische Wirkstoffe für Säugetiere, insbesondere für den Menschen, bei der Behandlung von kinasebedingten Krankheiten. Der Ausdruck "kinasebedingte Krankheiten " bezieht sich auf pathologische Zustände, die von der Aktivität einer oder mehrerer Proteinkinasen abhängig sind. Die Proteinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Proteinkinaseaktivität assoziiert sind, zählen Krebs, Tumorwachstum, Arteriosklerose, diabetischer Retinopathie und Entzündungserkrankungen.

Gewöhnlich werden die hier besprochenen Erkrankungen in zwei Gruppen eingeteilt, in hyperproliferative und nicht-hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht-krebsartige Krankheiten angesehen, von denen Arthritis, Entzündung, immunologische Krankheiten, Autoimmunkrankheiten und Immunschwächekrankheiten gewöhnlich als nicht-hyperproliferative Erkrankungen angesehen werden.

In diesem Zusammenhang sind Hirnkrebs, Lungenkrebs, Plattenepithelkrebs, Blasenkrebs, Magenkrebs, Pankreaskrebs, Leberkrebs, Nierenkrebs, Darmkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich zur Gruppe der hyperproliferative Erkrankungen gezählt werden. Insbesondere krebsartiges Zellwachstum und insbesondere durch PDK1 direkt oder indirekt vermitteltes krebsartiges Zellwachstum ist eine Erkrankung, die ein Ziel der vorliegenden Erfindung darstellt.

Gegenstand der vorliegenden Erfindung ist deshalb die Verwendung von erfindungsgemäßen Verbindungen zur Herstellung eines Medikaments für die Behandlung und/oder Prophylaxe der genannten Erkrankungen sowie auch ein Verfahren zur Behandlung der genannten Erkrankungen, umfassend die Verabreichung eines oder mehrerer erfindungsgemäßer Verbindungen an einen Patienten mit Bedarf an einer derartigen Verabreichung.

Der Empfänger oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Empfänglichkeit einer bestimmten Zelle gegenüber der Behandlung mit den erfindungsgemäßen Verbindungen kann durch *in vitro*-Tests bestimmt werden. Typischerweise wird eine Kultur der Zelle mit einer erfindungsgemäßen Verbindung bei verschiedenen Konzentrationen für eine Zeitdauer inkubiert, die ausreicht, um den Wirkstoffen zu ermöglichen, Zelltod zu induzieren oder Migration zu inhibieren, gewöhnlich zwischen ungefähr einer Stunde und einer Woche. Zu *in* vitro-Tests können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der spezifischen Zellzahl und kann fortgesetzt werden, bis im Wesentlichen keine unerwünschten Zellen mehr im Körper nachgewiesen werden.

Zur Identifikation von Kinase-Inhibitoren stehen verschiedene Assay-Systeme zur Verfügung. Beim Scintillation-Proximity-Assay (Sorg et al., J. of. Biomolecular Screening:7, 11-19, 2002) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 191-214, 2002).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., Biochem. J. 366:977-981, 2002).

Es gibt viele mit einer Deregulation der Zellproliferation und des Zelltods (Apoptose) einhergehende Erkrankungen und Krankheitszustände. Die Erkrankungen und Krankheitszustände die durch erfindungsgemäße Verbindungen behandelt, verhindert oder gelindert werden können umfassen die nachfolgend aufgeführten Erkrankungen und Krankheitszustände, sind jedoch nicht darauf beschränkt. Die erfindungsgemäßen Verbindungen sind nützlich bei der Behandlung und/oder Prophylaxe einer Reihe verschiedener Erkrankungen und Krankheitszustände, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die vorliegende Erfindung umfasst die Verwendung der erfindungsgemäßen Verbindungen zur Behandlung oder Vorbeugung von Krebs. Gegenstand der Erfindung ist insbesondere die Verwendung von erfindungsgemäßen Verbindungen und/oder ihrer physiologisch unbedenklichen Salze, Derivate, Solvate und Stereoisomere, einschließlich deren Mischungen in allen Verhältnissen zur Herstellung eines Medikaments zur Behandlung und/oder Prophylaxe von festen Tumoren, wobei der feste Tumor besonders bevorzugt aus der Gruppe bestehend aus Gehirntumor, Tumor des Urogenitaltrakts, Tumor des lymphatischen Systems, Magentumor, Kehlkopftumor, Lungentumor ausgewählt ist. Bevorzugt können auch feste Tumore ausgewählt aus der Gruppe bestehend aus Monozytenleukämie, Lungenadenokarzinom, kleinzellige und nicht- kleinzellige Lungenkarzinome, Nierenzellkarzinom, Endometriumkarzinom, multiples Myelom, Prostatakrebs, Kolorektalkrebs, Bauchspeicheldrüsenkrebs, Glioblastome und Brustkarzinom mit Medikamenten enthaltend erfindungsgemäße Verbindungen behandelt werden.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, zytotoxische Stoffe, antiproliferative Mittel, Prenyl-Proteintransferaseinhibitoren, HMG-CoA-Reduktase-Inhibitoren, HIV-Protease-Inhibitoren, Reverse-Transkriptase-Inhibitoren, Wachstumsfaktor-Inhibitoren sowie Angiogeneseinhibitoren. Die vorliegenden Verbindungen eignen sich insbesondere zur gemeinsamen Anwendung mit Radiotherapie.
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, wobei diese Aufzählung keine Einschränkung darstellen soll. "Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Inhibitoren, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxy-phenyl)retinamid und N-4-Carboxyphenylretinamid. "zytotoxische Stoffe" bezieht sich auf Verbindungen, die in erster Linie durch direkte Einwirkung auf die Zellfunktion zum Zelltod führen oder die die Zellmitose hemmen oder diese stören, darunter Alkylierungsmittel, Tumornekrosefaktoren, interkaliernde Mittel, Mikrotubulin-Inhibitoren und Topoisomerase-Inhibitoren.
Zu den zytotoxischen Stoffen zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.

Zu den Mikrotubulin-Inhibitoren zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Inhibitoren sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Amino-propylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch monoklonale Antikörper gegen Wachstumsfaktoren wie Erbitux, Trastuzumab, sowie Tumorsuppressorgene, wie p53, die über rekombinanten virusvermittelten Gentransfer abgegeben werden können (siehe z.B. US-Patent Nr. 6,069,134).

### Ausführungsbeispiele

Die nachfolgenden Beispiele dienen der Verdeutlichung der Erfindung. Die Erfindung ist nicht auf die Beispiele beschränkt. Auf der anderen Seite können Angaben aus den Beispielen, insbesondere zu den Reaktionsbedingungen, über die konkret beschriebenen Gegebenheiten hinaus allgemein im Rahmen der Erfindung anwendbar sein.

### Beispiel 1: Herstellung von Verbindungen der Formel AV

Es wird gemäß dem untenstehenden Schaubild folgendermaßen vorgegangen:
**1.1** Eine Mischung von Nitroanilin **1a** (1 Äquivalent) wird ohne Lösungsmittel für 10 Minuten bei 130 °C mit **2** (2 Äquivalente) umgesetzt. Aus dem entstehenden Rohprodukt wird **3a** durch Zugabe von Ethylacetat ausgefällt:
   4-(4-Methoxy-2-nitro-phenylamino)-2-methylsulfanyl-pyrimidin-5-carbonsäureethylester, 387 mg (43 %); gelbliches Pulver; HPLC: 2.73 min; LC-MS: 2.023, 365.0 m/z.
   Analog können folgende Verbindungen der Formel VI erhalten werden:
      3b) 4-(4,5-Dimethyl-2-nitro-phenylamino)-2-methylsulfanyl-pyrimidin-5-carbonsäureethylester: 335 mg (48 %); gelbliches Pulver; HPLC: 2.95 min; LC-MS: 2.27 min, 363.0 m/z.
      3c) 4-(4-Methyl-2-nitro-phenylamino)-2-methylsulfanyl-pyrimidin-5-carbonsäureethylester: 426 mg (49 %); gelbliches Pulver; HPLC: 2.85 min; LC-MS: 2.170 min, 349.0 m/z.
      3d) 4-(4-Chloro-2-nitro-phenylamino)-2-methylsulfanyl-pyrimidin-5-carbonsäureethylester: 1585 mg (72 %); gelbliches Pulver; HPLC: 2.95 min; LC-MS: 2.606 min, 369.0 m/z.
      3e) 4-(5-Methyl-2-nitro-phenylamino)-2-methylsulfanyl-pyrimidin-5-carbonsäureethylester: 784 mg (75 %); gelbliches Pulver; HPLC: 3.03 min; LC-MS: 2.652 min, 349.0 m/z.
      3f) 4-(4-Methoxy-2-nitro-phenylamino)-2-methylsulfanyl-pyrimidine-5-carbonsäureethylester: 387 mg (43 %); gelbliches Pulver; HPLC: 2.73 min; LC-MS: 2.023 min, 365.0 m/z.
      3g) 4-(4-Ethoxy-2-nitro-phenylamino)-2-methylsulfanyl-pyrimidine-5-carbonsäureethylester
      3h) 2-Methylsulfanyl-4-(3-nitro-pyridin-2-ylamino)-pyrimidine-5-carbonsäureethylester
      3i) 4-(4-Bromo-2-nitro-phenylamino)-2-methylsulfanyl-pyrimidine-5-carbonsäureethylester
      3j) 2-Methylsulfanyl-4-(4-methyl-3-nitro-pyridin-2-ylamino)-pyrimidine-5-carbonsäureethylester
**1.2 3a** wird in THF gelöst und unter Zuhilfenahme von Pd/C als Katalysator und Wasserstoff bei Raumtemperatur und Normaldruck über 24 h zu **4a** reduziert. Der Katalysator wird abfiltriert, mit THF nachgewaschen und das gewünschte Produkt durch Abdestillieren des Lösungsmittels im Vakuum erhalten:
   4-(2-Amino-4-methoxy-phenylamino)-2-methylsulfanyl-pyrimidin-5-carbonsäureethylester: 874 mg (100 %); gelbes Pulver; HPLC: 2.27 min; LC-MS: 1.38 min, 335.0 m/z.
      Analog können folgende Verbindungen der Formel Verhalten werden:
      4b) 4-(2-Amino-4,5-dimethyl-phenylamino)-2-methylsulfanyl-pyrimidine-5-carbonsäureethylester: 640 mg (100 %); gelbes Pulver; HPLC: 2.28 min; LC-MS: 1.373 min, 333.0 m/z.
      4c) 4-(2-Amino-4-methyl-phenylamino)-2-methylsulfanyl-pyrimidine-5-carbonsäureethylester: 686 mg (93 %); gelbes Pulver; HPLC: 2.27 min; LC-MS: 1.41 min, 319.0 m/z.
      4d) 4-(2-Amino-4-chloro-phenylamino)-2-methylsulfanyl-pyrimidine-5-carbonsäureethylester: 770 mg (97 %); gelbes Pulver; HPLC: 2.51 min; LC-MS: 2.79 min, 339.0 m/z.
      4e) 4-(2-Amino-5-methyl-phenylamino)-2-methylsulfanyl-pyrimidine-5-carbonsäureethylester: 771 mg (98 %); gelbes Pulver; HPLC: 2.23 min; LC-MS: 1.29 min, 319.0 m/z.
      4f) 4-(2-Amino-4-methoxy-phenylamino)-2-methylsulfanyl-pyrimidine-5-carbonsäureethylester: 874 mg (100 %); gelbes Pulver; HPLC: 2.27 min; LC-MS: 1.38 min, 335.0 m/z.
      4g) 4-(2-Amino-4-ethoxy-phenylamino)-2-methylsulfanyl-pyrimidine-5-carbonsäureethylester
      4h) 4-(3-Amino-pyridin-2-ylamino)-2-methylsulfanyl-pyrimidine-5-carbonsäureethylester
      4i) 4-(2-Amino-4-bromo-phenylamino)-2-methylsulfanyl-pyrimidine-5-carbonsäureethylester
**1.3 4a** wird ohne weitere Aufreinigung mit 1.5 Äquivalenten Natronlauge in Dioxan (10 ml / g) bei 100 °C (30 Min) in einem Mikrowellenofen verseift. Das gewünschte Produkt **(5a)** wird durch Zugabe von Salzsäure ausgefällt, abgesaugt, mit wenig Wasser nachgewaschen und getrocknet: 4-(2-Amino-4-methoxy-phenylamino)-2-methylsulfanyl-pyrimidin-5-carbonsäure: 450 mg (98 %); gebrochen weißes Pulver; HPLC: 2.08 min; LC-MS: 0.814 min, 307.0 m/z.
   Analog können folgende Verbindungen der Formel IV erhalten werden:
   5b) 4-(2-Amino-4,5-dimethyl-phenylamino)-2-methylsulfanyl-pyrimidine-5-carbonsäure: 911 mg (98 %); gebrochen weißes Pulver; HPLC: 2.12 min; LC-MS: 1.523 min, 305.0 m/z.
   5c) 4-(2-Amino-4-methyl-phenylamino)-2-methylsulfanyl-pyrimidine-5-carbonsäure: 625 mg (92 %); gebrochen weißes Pulver; HPLC: 2.09 min; LC-MS: 1.440 min, 291.0 m/z.
   5d) 4-(2-Amino-4-chloro-phenylamino)-2-methylsulfanyl-pyrimidine-5-carbonsäure: 591 mg (84 %); gebrochen weißes Pulver; HPLC: 2.31 min; LC-MS: 1.27 min, 311.0 m/z.
   5e) 4-(2-Amino-5-methyl-phenylamino)-2-methylsulfanyl-pyrimidine-5-carbonsäure: 619 mg (100 %); gebrochen weißes Pulver; HPLC: 2.07 min; LC-MS: 0.796 min, 291.0 m/z.
   5f) 4-(2-Amino-4-methoxy-phenylamino)-2-methylsulfanyl-pyrimidine-5-carbonsäure: 450 mg (98 %); gebrochen weißes Pulver; HPLC: 2.08 min; LC-MS: 0.814 min, 307.0 m/z.
   5g) 4-(2-Amino-4-ethoxy-phenylamino)-2-methylsulfanyl-pyrimidine-5-carbonsäure
   5h) 4-(3-Amino-pyridin-2-ylamino)-2-methylsulfanyl-pyrimidine-5-carbonsäure
   5i) 4-(2-Amino-4-bromo-phenylamino)-2-methylsulfanyl-pyrimidine-5-carbonsäure
**1.4 5a** wird unter Zuhilfenahme von 1.2 Äquivalenten N-(3-Dimethylaminopropyl)-N'-ethylcarbodiimid-Hydrochlorid, 1.2 Äquivalenten HOBt und 2.2 Äquivalenten 4-Methylmorpholin zu **6a** cyclisiert und das gewünschte Produkt nach Extraktion mit Ethylacetat/nButanol säulenchromatographisch mit einem Flash-Master II (siehe unten) aufgereinigt: 8-Methoxy-3-methylsulfanyl-5,10-dihydro-2,4,5,10-tetraazadibenzo[a,d]cyclohepten-11-on: 263 mg (56 %); gelbes Pulver; HPLC: 2.21 min; LC-MS: 1.01 min, 289.0 m/z.
   Analog können folgende Verbindungen der Formel III erhalten werden:
   6b) 7,8-Dimethyl-3-methylsulfanyl-5,10-dihydro-2,4,5,10-tetraazadibenzo[a,d]cyclohepten-11-on: 800 mg (93 %); gelbes Pulver; HPLC: 2.31 min; LC-MS: 1.769 min, 287.0 m/z.
   6c) 8-Methyl-3-methylsulfanyl-5,10-dihydro-2,4,5,10-tetraazadibenzo[a,d]cyclohepten-11-on: 311 mg (72 %); gelbes Pulver; HPLC: 2.24 min; LC-MS: 1.158 min, 273.0 m/z.
   6d) 8-Chloro-3-methylsulfanyl-5,10-dihydro-2,4,5,10-tetraazadibenzo[a,d]cyclohepten-11-on: 108 mg (20 %); gelbes Pulver; HPLC: 2.38 min; LC-MS: 1.33 min, 293.0 m/z.
   6e) 7-Methyl-3-methylsulfanyl-5,10-dihydro-2,4,5,10-tetraazadibenzo[a,d]cyclohepten-11-on: 617 mg (94 %); gelbes Pulver; HPLC: 2.25 min; LC-MS: 1.189 min, 273.0 m/z.
   6f) 8-Methoxy-3-methylsulfanyl-5,10-dihydro-2,4,5,10-tetraazadibenzo[a,d]cyclohepten-11-on: 263 mg (56 %); gelbes Pulver; HPLC: 2.21 min; LC-MS: 1.01 min, 289.0 m/z.
   6g) 8-Ethoxy-3-methylsulfanyl-5,10-dihydro-2,4,5,10-tetraazadibenzo[a,d]cyclohepten-11-on
   6h) 3-Methylsulfanyl-5,10-dihydro-2,4,5,6,10-pentaazadibenzo[a,d]cyclohepten-11-on
   6i) 8-Bromo-3-methylsulfanyl-5,10-dihydro-2,4,5,10-tetraazadibenzo[a,d]cyclohepten-11-on
**1.5** Zur Vorbereitung der Substitution wird **6a** mit 4 Äquivalenten meta-Chlorperbenzoesäure in einem THF / Dichlormethan-Gemisch (Verhältnis 1:1) behandelt (15 Min 0 °C, 2 h RT). Man erhält so nach Zugabe von 20 %iger Natriumsulfit-Lösung einen Niederschlag, der abgesaugt und mit wenig Wasser nachgewaschen wird. Dieser Niederschlag enthält zu etwa 58 % (laut HPLC) das Oxidationsprodukt **7a.** Selbiger wird ohne weitere Aufreinigung mit 1.2 Äquivalenten Methyl-(1-methyl-piperidin-4-yl)-amin **A** unter Zugabe von 0.1 Äquivalente Kaliumiodid und 1.5 Äquivalenten Kaliumcarbonat für 30 Minuten auf 100 °C erhitzt. Das gewünschte Produkt 8a kann nach Filtration und Konzentration im Vakuum säulenchromatographisch mittels präparativer HPLC aufgereinigt werden: 8-Methoxy-3-(1-methyl-piperidin-4-ylamino)-5,10-dihydro-2,4,5,10-tetraazadibenzo[a,d]cyclohepten-11-on: 23 mg (100 %); orange-brauner Feststoff; HPLC: 2.01 min; LC-MS: 0.458 min, 355.2 m/z.
   Analog können folgende Zwischenprodukte erhalten werden:
   8b) 7,8-Dimethyl-3-[methyl-(1-methyl-piperidin-4-yl)-amino]-5,10-dihydro-2,4,5,10-tetraaza-dibenzo[a,d]cyclohepten-11-on: 11 mg (3 % d.Th.); orange-brauner Feststoff; HPLC: 2.06 min; LC-MS: 1.387 min, 367.2 m/z.
   8c) 8-Chloro-3-[methyl-(1-methyl-piperidin-4-yl)-amino]-5,10-dihydro-2,4,5,10-tetraaza-dibenzo[a,d]cyclohepten-11-on: 58 mg (47 % d.Th.); orange-brauner Feststoff; HPLC: 2.08 min; LC-MS: 0.678 min, 373.0 m/z.
   8d) 8-Methoxy-3-[methyl-(1-methyl-piperidin-4-yl)-amino]-5,10-dihydro-2,4,5,10-tetraaza-dibenzo[a,d]cyclohepten-11-on: 31 mg (9 % d.Th.); orange-brauner Feststoff; HPLC: 2.01 min; LC-MS: 1.229 min, 369.2 m/z.
   8e) 8-Methyl-3-[methyl-(1-methyl-piperidin-4-yl)-amino]-5,10-dihydro-2,4,5,10-tetraaza-dibenzo[a,d]cyclohepten-11-on: 20 mg (5 % d.Th.); orange-brauner Feststoff; HPLC: 2.02 min; LC-MS: 1.275 min, 353.2 m/z.
   8f) 8-Methoxy-3-(1-methyl-piperidin-4-ylamino)-5,10-dihydro-2,4,5,10-tetraaza-dibenzo[a,d]cyclohepten-11-on: 23 mg (100 % d.Th.); orange-brauner Feststoff; HPLC: 2.01 min; LC-MS: 0.458 min, 355.2 m/z.
   8g) 7-Methyl-3-[methyl-(1-methyl-piperidin-4-yl)-amino]-5,10-dihydro-2,4,5,10-tetraaza-dibenzo[a,d]cyclohepten-11-on: 13 mg (9 % d.Th.); orange-brauner Feststoff; HPLC: 1.92 min; LC-MS: 1.273 min, 353.2 m/z.
   8h) 7-Methyl-3-(1-methyl-piperidin-4-ylamino)-5,10-dihydro-2,4,5,10-tetraaza-dibenzo[a,d]cyclohepten-11-on: 5 mg (2 % d.Th.); orange-brauner Feststoff; HPLC: 1.96 min; LC-MS: 1.253 min, 339.2 m/z.
   8i) 7,8-Dimethyl-3-(1-methyl-piperidin-4-ylamino)-5,10-dihydro-2,4,5,10-tetraaza-dibenzo[a,d]cyclohepten-11-on: 70 mg (21 % d.Th.); orange-brauner Feststoff; HPLC: 1.96 min; LC-MS: 1.338 min, 353.2 m/z.

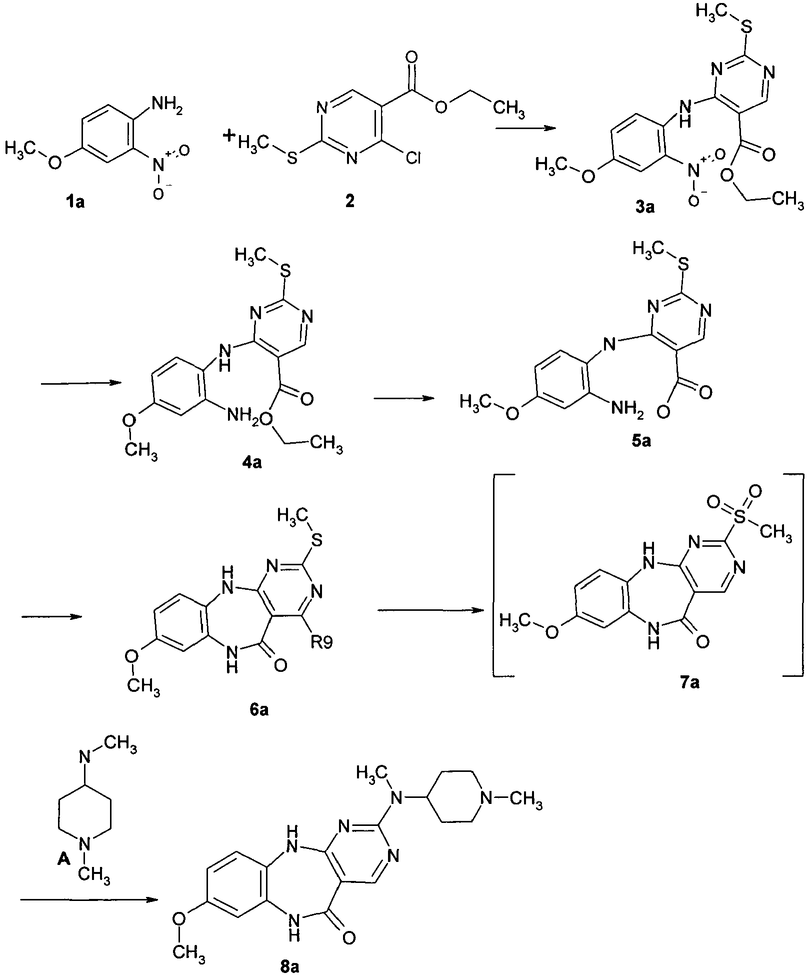

Es werden folgende Geräte verwendet:

Die Säulenchromatographie wird mit dem Flash-Master II (Biotage, Schweden) durchgeführt. Die Kunststoffkartuschen werden mit Kieselgel der Korngröße 0.003-0.006 mm (Merck Eurolab, Darmstadt) befüllt.

Die präperative HPLC wird mit einer Chromolith Prep (RP-18e 100-25 mm) Säule, einer K-1800 Gradient Pumpe und einem Büchi Fraction Collector B684 durchgeführt. Für die Trennung wird ein Gemisch von Wasser / 0.1 % TFA (Laufmittel A) und Acetonitril / 0.1% TFA (Laufmittel B) bei einer Flussrate von 30 ml/min eingesetzt.

Analytische HPLC: Die HPLC-Spektren werden mit einer Lichrograph L-6200A Gradienten-Pumpe, einem L-4500A Diodenarray Detektor und einer Chromolith Speed ROD RP-18e 50-4.6 mm Säule (alles Merck, Darmstadt) und mit Hilfe des Computerprogramms D-6500 DAD System Manager Revision 1 aufgenommen und bearbeitet. Die HPLC-Reinheiten werden mittels UV-Detektion bei□ 220 nm gemessen. Für die Reinheitsbestimmungen wird ein Gradient von Wasser / 0.1 % TFA (Laufmittel A) und Acetonitril / 0.1 % TFA (Laufmittel B) bei einer Flussrate von 3 ml/min und einer Laufzeit von 5 min verwendet. Die in der Tabelle 1 mit (1) gekennzeichneten HPLC-Daten wurden mit dieser Methode erhalten. Bei den mit (2) gekennzeichneten HPLC-Daten wurde eine Flussrate von 1,5 ml/min und eine Laufzeit von 6 min angewandt. Die mit einem Stern gekennzeichneten zweifach bestimmten Retentionszeiten weisen auf das Vorliegen von Konformeren hin.

Die HPLC-MS Spektren werden mit dem Agilent System 1100 und einer Chromolith Speed ROD RP-18e 50-4.6 mm Säule aufgenommen und gemessen. Für die Trennungen wurde ein Gradient von Wasser / 0.1 % TFA (Laufmittel A) und Acetonitril / 0.1 % TFA (Laufmittel B) bei einer Flussrate von 2.4 ml/min verwendet. Die in der Tabelle 1 angegebenen HPLC-MS-Daten wurden mit dieser Methode erhalten.

### Beispiel 2: Hemmung von PDK1 (IC₅₀)

Der Kinaseassay kann als 384-well Flashplate-Assay durchgeführt werden. 3.4 nM His6-PDK1(Δ1-50), 400 nM PDKtide (Biotin-bA-bA-KTFCGTPEYL-APEVRREPRILSEEEQEMFRDFDYIADWC) und 4 µM ATP (mit 0.2 µCi 33P-ATP/Well) werden in einem Gesamtvolumen von 50µl (50 mM TRIS, 10 mM Mg-acetat, 0.1 % Mercaptoethanol, 0.02 % Brij35, 0.1 % BSA, pH 7.5) ohne oder mit Prüfsubstanz (5-10 Konzentrationen) für 60 Min bei 30 °C inkubiert. Die Reaktion wird mit 25 µl 200 mM EDTA-Lösung gestoppt, nach 30 Min bei Raumtemperatur abgesaugt und die Wells mit 3mal 100 µl 0.9%-iger NaCl-Lösung gewaschen. Der unspezifische Anteil der Kinasereaktion (Blank) wird mit 100 nM Staurosporine bestimmt. Radioaktivität wird mit einem Topcount Szintillationszähler (PerkinElmer, USA) gemessen. IC50-Werte werden mit dem Computerprogramm RS1 berechnet.

Weitere Inhibitionskonstanten von erfindungsgemäßen Verbindungen sind in der Tabelle 1 aufgeführt.

Die nachfolgenden Beispiele betreffen pharmazeutische Zubereitungen:

### Beispiel 3a: Injektionsgläser

Eine Lösung von 100 g eines erfindungsgemäßen Wirkstoffes und 5 g Dinatriumhydrogenphosphat wird in 3 L zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel 3b: Suppositorien

Man schmilzt ein Gemisch von 20 g eines erfindungsgemäßen Wirkstoffes mit 100 g Sojalecithin und 1400 g Kakaobutter, gießt in Formen und lässt erkalten. Jedes Suppositorium enthält 20 mg Wirkstoff.

### Beispiel 3c: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 mL zweifach destilliertem Wasser. Man stellt auf pH 6,8 ein, füllt auf 1 L auf und sterilisiert durch Bestrahlung. Diese Lösung kann in Form von Augentropfen verwendet werden.

### Beispiel 3d: Salbe

Man mischt 500 mg eines erfindungsgemäßen Wirkstoffes mit 99,5 g Vaseline unter aseptischen Bedingungen.

### Beispiel 3e: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel 3f: Dragees

Analog Beispiel 5e werden Tabletten gepresst, die anschließend in üblicher Weise mit einem Überzug aus Saccharose, Kartoffelstärke, Talk, Tragant und Farbstoff überzogen werden.

### Beispiel 3g: Kapseln

2 kg Wirkstoff werden in üblicher Weise in Hartgelatinekapseln gefüllt, so dass jede Kapsel 20 mg des Wirkstoffs enthält.

### Beispiel 3h: Ampullen

Eine Lösung von 1 kg eines erfindungsgemäßen Wirkstoffes in 60 L zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel AV 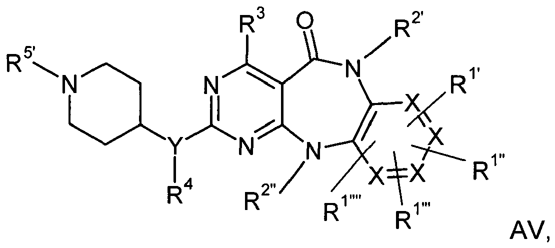 worin
R^{1'}, R^{1"}, R^{1"'}, R^{1""}, R³, R⁴, R^{5'}, jeweils unabhängig voneinander H, A, R⁶ ,Ar, OR⁶, SR⁶, OAr, SAr, N(R⁶)₂, NHAr, Hal, NO₂, CN, (CH₂)ₘCOOR⁶, (CH₂)ₘCOOAr, (CH₂)ₘCON(R⁶)₂, (CH₂)ₘCONAAr, COA, COR⁶, COAr, S(O)ₘA, S(O)ₘAr, NACOA, NACOAr, NASO₂A, NASO₂Ar, NHCOA, NHCOAr, NHCON(R⁶)₂, NHCONHA, NHCONHAr, SO₂N(R⁶)₂, SO₂NAAr, M(CH₂)ₙN(R⁶)₂, M(CH₂)ₙNAR⁶, M(CH₂)ₙNA₂, M(CH₂)ₙ(R⁶)ₙ, M(CH₂)ₙ(R⁶)ₙ, M(CH₂)ₙ(R⁶)ₙ, M(CH₂)ₙ(R⁶)ₙ, M(CH₂)ₙ-oxopiperazin, M(CH₂)ₙ-oxomorpholin, M(CH₂)ₙ-oxopyrrolidin, M(CH₂)ₙC(CH₃)ₙ(CH₂)ₙN(R⁶)₂, M(CH₂)ₙM(R⁶)ₙSOₘA, M(CH₂)ₙM(R⁶)ₙSOₘM(R⁶)ₙ, M(CH₂)ₙM(R⁶)ₙSOₘAr, (CH₂)ₙM(R⁶)ₙSOₘA, (CH₂)ₙM(R⁶)ₙSOₘM(R⁶)ₙ, (CH₂)ₙM(R⁶)ₙSOₘAr, M(CH₂)ₙSOₘA, M(CH₂)ₙSOₘN(R⁶)ₙA, M(CH₂)ₙSOₘAr, (CH₂)ₙSOₘA, (CH₂)ₙSOₘM(R₆)ₙ, (CH₂)ₙSOₘAr, wobei zwei benachbart stehende Reste R^{1'}, R^{1"}, R^{1'"} oder R^{1""}, miteinander einen gesättigten oder ungesättigten, optional ein-oder zweifach durch M substituierten, fünf- oder sechsgliedrigen Carbo- oder Heterozyklus bilden können,
R^{2'}, R^{2"} jeweils unabhängig voneinander R⁶,
R⁶ H, Hal, OH, CN, NH₂, NO₂, SO₂, unverzweigtes oder verzweigtes Alkyl mit 1-4 C-Atomen, worin eine CH₂-Gruppe durch ein O oder S-Atom und/oder durch eine NH, NA, CONH, NHCO oder -CH=CH- Gruppe und/oder auch 1-4 H-Atome durch Hal ersetzt sein können, und worin eine CH₃-Gruppe durch Hal, OH, CN, NH₂, NHR₇, NR⁷₂, NO₂ oder SO₂ ersetzt sein kann, wobei R⁷ = Methyl oder Ethyl bedeutet, wobei zwei Reste R⁶ zusammen mit dem Atom, mit dem sie veknüpft sind, einen gesättigten oder ungesättigten fünf- oder sechsgliedrigen Carbo- oder Heterozyklus bilden können,
n 0, 1, 2, 3, 4 oder 5,
m 0, 1 oder 2,
A unverzweigtes, verzweigtes oder cyclisches Alkyl mit 1-14 C-Atomen, worin eine oder zwei CH₂-Gruppen durch ein O- oder S-Atom und/oder durch eine NH, CONH, NHCO, CO oder - CH=CH- Gruppe und/oder auch 1-7 H-Atome durch Hal ersetzt sein können, und worin eine oder zwei CH₃-Gruppen durch R⁶ ersetzt sein können,
Ar einen ein- oder zweikernigen aromatischen Homo- oder Heterocyclus mit 1 bis 4 N-, O- und/oder S-Atomen und 5 bis 10 Gerüstatomen, der unsubstituiert oder ein-, zwei- oder dreifach durch Carbonylsauerstoff, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ und/oder S(O)ₘA substituiert sein kann,
Hal F, Cl, Br oder I,
X CR¹ ist, worin R¹ stellvertretend für einen der Reste R^{1'}, R^{1"}, R^{1"'} oder R^{1""} steht,
Y NR⁴, O oder S, und
M NH, O, S
bedeuten,
sowie ihre pharmazeutisch unbedenklichen Salze, Solvate und Stereoisomere, einschließlich deren Mischungen in allen Verhältnissen.

2. Verbindungen nach Anspruch 1, worin bei der Teilformel Ac
R^{5'} Methyl bedeutet,
bei der Teilformel Ad
R³ H bedeutet,
bei der Teilformel Ae
R^{2'}, R^{2"} H bedeuten,
bei der Teilformel Af
Y NR⁴,
R⁴ H oder Methyl bedeutet,
bei der Teilformel Ag
R^{1'}, R^{1"} H,
R^{1'"} H, Hal oder Methyl,
R^{1""} H, Hal, Methyl, Ethyl, n-Propyl, 2-Propyl, Butyl, Isobutyl, sek.-Butyl tert.-Butyl, Methoxy, CHal₃, CF₃, OH, OCH₂CH₂OH, SCH₂CH₃, NHCH₃, N(CH₃)₂, CN, COOH, COOCH₃, SO₂OH, OCHal₃, OCF₃, NHCOA, NHCOAr, NHCON(R⁶)₂, NHCONHA, NHCONHAr bedeutet, wobei A und R⁶ H, Cyclopentyl, Cyclo hexyl, n-Propyl, 2-Propyl, Ethyl, sek.-Butyl oder tert.-Butyl sind und Ar Thiophen-2 oder 3-yl, 3,5-Dimethyl-isoxazol-4-yl ist, und zwei Reste R⁶ gemeinsam mit dem Amid-Stickstoffatom einen Tetrahydropyrrol-Ring bilden können,
bei der Teilformel Ah
Y NR⁴,
R⁴ H oder Methyl,
R^{5'} Methyl,
R^{2'}, R^{2"}, R³ H,
R^{1'}, R^{1"} H,
R^{1'"} H, Hal oder Methyl,
R^{1""} H, Hal, Methyl, Ethyl, n-Propyl, 2-Propyl, Butyl, Isobutyl, sek.-Butyl tert.-Butyl, Methoxy, CHal₃, CF₃, OH, OCH₂CH₂OH, SCH₂CH₃, NHCH₃, N(CH₃)₂, CN, COOH, COOCH₃, SO₂OH, OCHal₃, OCF₃, NHCOA, NHCOAr, NHCON(R⁶)₂, NHCONHA, NHCONHAr bedeutet, wobei A und R⁶ H, Cyclopentyl, Cyclo hexyl, n-Propyl, 2-Propyl, Ethyl, sek.-Butyl oder tert.-Butyl sind und Ar Thiophen-2 oder 3-yl, 3,5-Dimethyl-isoxazol-4-yl ist, und zwei Reste R⁶ gemeinsam mit dem Amid-Stickstoffatom einen Tetrahydropyrrol-Ring bilden können,
sowie ihre pharmazeutisch unbedenklichen Salze, Solvate und Stereoisomere, einschließlich deren Mischungen in allen Verhältnissen.

3. Verfahren zur Herstellung von Verbindungen der Formel AV sowie ihrer physiologisch unbedenklichen Salze, Solvate und Stereoisomere, **dadurch gekennzeichnet,**
**dass** man eine Verbindung der Formel VIII, 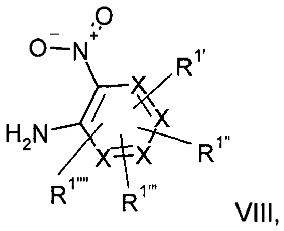 worin sämtliche Reste die zuvor angegebene Bedeutung haben mit einer Verbindung der Formel VII 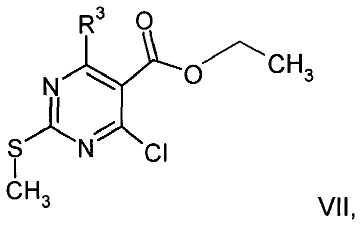 worin sämtliche Reste die zuvor angegebene Bedeutungen haben, zu einer Verbindung der Formel VI umsetzt 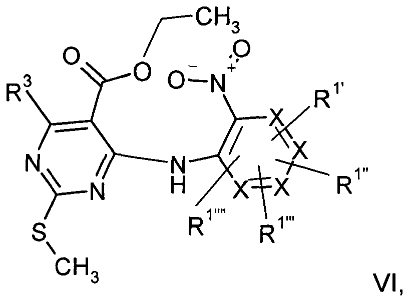 die zu einer Verbindung der Formel V 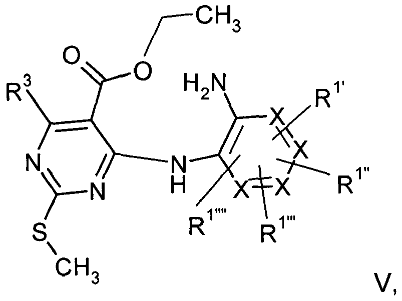 reduziert wird, welche dann im nächsten Schritt zu einer Verbindung der Formel IV 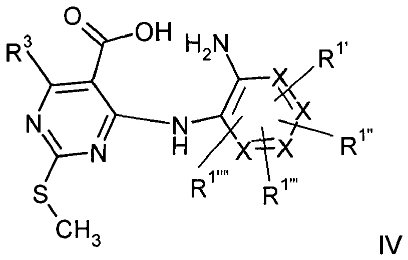 verseift wird, die weiter zu einem Diazepinon der Formel III 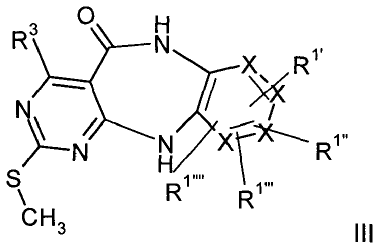 Oxidation zu einem Sulfon, mit einer Verbindung der Formel II 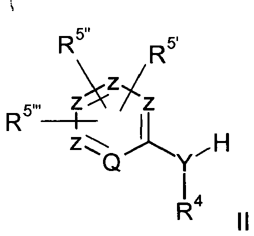 substituiert wird, wobei eine Verbindung der Formel Ib 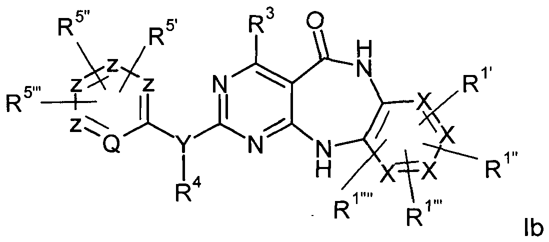 erhalten wird, die schließlich, falls die Reste R^{2'}, R^{2"} eine andere Bedeutung als H haben, zu einer Verbindung der Formel AV umgesetzt und ggf. eine Base oder Säure der Formel AV in eines ihrer Salze umgewandelt wird.

4. Verbindungen nach einem oder mehreren der Ansprüche 1 bis 3 und/oder ihre physiologisch unbedenklichen Salze, Solvate und Stereoisomere, einschließlich deren Mischungen in allen Verhältnissen zur Verwendung als Arzneimittel.

5. Arzneimittel, enthaltend wenigstens eine Verbindung nach einem oder mehreren der Ansprüche 1 bis 3 und/oder ihre physiologisch unbedenklichen Salze, Solvate und Stereoisomere, einschließlich deren Mischungen in allen Verhältnissen, sowie gegebenenfalls Träger- und/oder Hilfsstoffe.

6. Arzneimittel, enthaltend wenigstens eine Verbindung nach einem oder mehreren der Ansprüche 1 bis 3 und/oder ihre physiologisch unbedenklichen Salze, Solvate und Stereoisomere, einschließlich deren Mischungen in allen Verhältnissen sowie wenigstens einen weiteren Arzneimittelwirkstoff.

7. Set (Kit) bestehend aus getrennten Packungen von
a) einer wirksamen Menge einer Verbindung nach einem oder mehreren der Ansprüche 1 bis 3 und/oder ihrer physiologisch unbedenklichen Salze, Solvate und Stereoisomere, einschließlich deren Mischungen in allen Verhältnissen und
b) einer wirksamen Menge eines weiteren Arzneimittelwirkstoffs.

8. Verbindungen nach einem oder mehreren der Ansprüche 1 bis 3 sowie ihre physiologisch unbedenklichen Salze, Solvate und Stereoisomere, einschließlich deren Mischungen in allen Verhältnissen zur Verwendung als Aktivatoren oder Inhibitoren von Kinasen, insbesondere Tyronsin-Kinasen.

9. Verbindungen nach einem oder mehreren der Ansprüche 1 bis 3 sowie ihre physiologisch unbedenklichen Salze, Solvate und Stereoisomere, einschließlich deren Mischungen in allen Verhältnissen zur Verwendung als Inhibitoren der Serin/Threonin-Kinase PDK1.

10. Verwendung von Verbindungen nach einem oder mehreren der Ansprüche 1 bis 3 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Krankheiten, bei denen die Hemmung der Serin/Threonin-Kinase PDK1 zur Verbesserung des Krankheitsbildes führt.

11. Verwendung von Verbindungen nach einem oder mehreren der Ansprüche 1 bis 3 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Krebs, Tumorwachstum, Tumorangiogenese, Arteriosklerose, der diabetischen Retinopathie und Entzündungserkrankungen.

12. Verwendung von Verbindungen nach einem oder mehreren der Ansprüche 1 bis 3 und/oder ihrer physiologisch unbedenklichen Salze, Solvate und Stereoisomere, einschließlich deren Mischungen in allen Verhältnissen zur Herstellung eines Medikaments zur Prophylaxe oder Behandlung von Brustkrebs, Prostatakrebs, Kolorektalkrebs, kleinzelligem Lungenkrebs, nicht-kleinzelligem Lungenkrebs, multiplem Myelom sowie dem Nierenzellkarzinom, Glioblastom und dem Endometriumkarzinom.

13. Verbindung nach Anspruch 1 ausgewählt aus der Gruppe bestehend aus 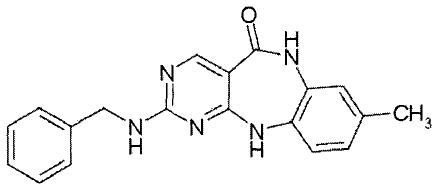 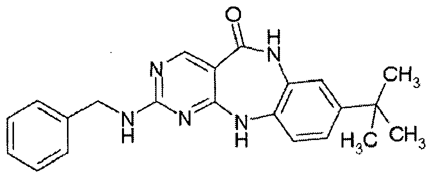 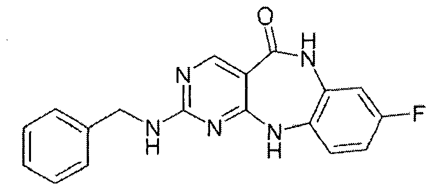 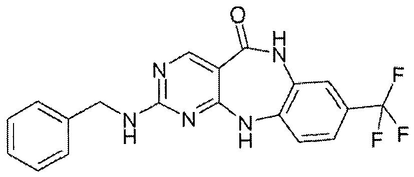 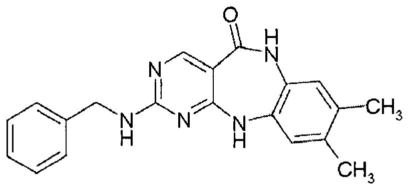 sowie ihre pharmazeutisch unbedenklichen Salze, Solvate und Stereoisomere, einschließlich deren Mischungen in allen Verhältnissen.

## Claims

1. Compounds of the formula AV 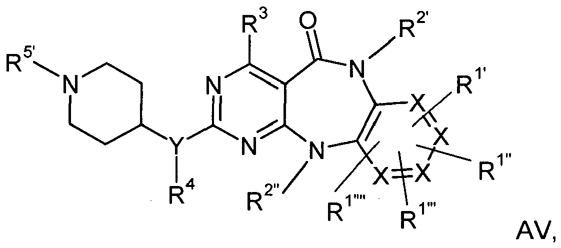 in which
R^{1'}, R^{1"}, R^{1"'}, R^{1""}, R³, R⁴, R^{5'} each, independently of one another, denote H, A, R⁶, Ar, OR⁶, SR⁶, OAr, SAr, N(R⁶)₂, NHAr, Hal, NO₂, CN, (CH₂)ₘCOOR⁶, (CH₂)ₘCOOAr, (CH₂)ₘCON(R⁶)₂, (CH₂)ₘCONAAr, COA, COR⁶, COAr, S(O)ₘA, S(O)ₘAr, NACOA, NACOAr, NASO₂A, NASO₂Ar, NHCOA, NHCOAr, NHCON(R⁶)₂, NHCONHA, NHCONHAr, SO₂N(R⁶)₂, SO₂NAAr, M(CH₂)ₙN(R⁶)₂, M(CH₂)ₙNAR⁶, M(CH₂)ₙNA₂, M(CH₂)ₙ(R⁶)ₙ, M(CH₂)ₙ(R⁶)ₙ, M(CH₂)ₙ(R⁶)ₙ, M(CH₂)ₙ(R⁶)ₙ, M(CH₂)ₙ-oxopiperazine, M(CH₂)ₙ-oxomorpholine, M(CH₂)ₙ-oxopyrrolidine, M(CH₂)ₙC(CH₃)ₙ(CH₂)ₙN(R⁶)₂, M(CH₂)ₙM(R⁶)ₙSOₘA, M(CH₂)ₙM(R⁶)ₙSOₘM(R⁶)ₙ, M(CH₂)ₙM(R⁶)ₙSOₘAr, (CH₂)ₙM(R⁶)ₙSOₘA, (CH₂)ₙM(R⁶)ₙSOₘM(R⁶)ₙ, (CH₂)ₙM(R⁶)ₙSOₘAr, M(CH₂)ₙSOₘA, M(CH₂)ₙSOₘN(R⁶)ₙA, M(CH₂)ₙSOₘAr, (CH₂)ₙSOₘA, (CH₂)ₙSOₘM(R⁶)ₙ, (CH₂)ₙSOₘAr, where two adjacent radicals R^{1'}, R^{1"}, R^{1"'} or R^{1""} may form a saturated or unsaturated, five- or six-membered carbo- or heterocycle which is optionally mono- or disubstituted by M with one another,
R^{2'}, R^{2"} each, independently of one another, denote R⁶,
R⁶ denotes H, Hal, OH, CN, NH₂, NO₂, SO₂, unbranched or branched alkyl having 1-4 C atoms, in which one CH₂ group may be replaced by an O or S atom and/or by an NH, NA, CONH, NHCO or -CH=CH- group and/or, in addition, 1-4 H atoms may be replaced by Hal, and in which one CH₃ group may be replaced by Hal, OH, CN, NH₂, NHR₇, NR⁷₂, NO₂ or SO₂, where R⁷ = methyl or ethyl, where two radicals R⁶, together with the atom to which they are linked, can form a saturated or unsaturated five- or six-membered carbo- or heterocycle,
n denotes 0, 1, 2, 3, 4 or 5,
m denotes 0, 1 or 2,
A denotes unbranched, branched or cyclic alkyl having 1-14 C atoms, in which one or two CH₂ groups may be replaced by an O or S atom and/or by an NH, CONH, NHCO, CO or -CH=CH-group and/or, in addition, 1-7 H atoms may be replaced by Hal, and in which one or two CH₃ groups may be replaced by R⁶,
Ar denotes a mono- or bicyclic aromatic homo- or heterocycle having 1 to 4 N, O and/or S atoms and 5 to 10 skeleton atoms, which may be unsubstituted or mono-, di- or trisubstituted by carbonyl oxygen, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ and/or S(O)ₘA,
Hal denotes F, Cl, Br or I,
X is CR¹, in which R¹ is representative of one of the radicals R^{1'}, R^{1"}, R^{1'"} or R^{1""}
Y denotes NR⁴, O or S, and
M denotes NH, O, S,
and pharmaceutically acceptable salts, solvates and stereoisomers thereof, including mixtures thereof in all ratios.

2. Compounds according to Claim 1 in which
in the sub-formula Ac
R^{5'} denotes methyl,
in the sub-formula Ad
R³ denotes H,
in the sub-formula Ae
R^{2'}, R^{2"} denote H,
in the sub-formula Af
Y denotes NR⁴,
R⁴ denotes H or methyl,
in the sub-formula Ag
R^{1'}, R^{1"} denote H,
R¹ denotes H, Hal or methyl,
R^{1""} denotes H, Hal, methyl, ethyl, n-propyl, 2-propyl, butyl, isobutyl, sec-butyl, tert-butyl, methoxy, CHal₃, CF₃, OH, OCH₂CH₂OH, SCH₂CH₃, NHCH₃, N(CH₃)₂, CN, COOH, COOCH₃, SO₂OH, OCHal₃, OCF₃, NHCOA, NHCOAr, NHCON(R⁶)₂, NHCONHA, NHCONHAr, where A and R⁶ are H, cyclopentyl, cyclohexyl, n-propyl, 2-propyl, ethyl, sec-butyl or tert-butyl and Ar is thio-phen-2- or -3-yl, 3,5-dimethylisoxazol-4-yl, and two radicals R⁶, together with the amide nitrogen atom, can form a tetrahydro-pyrrole ring,
in the sub-formula Ah
Y denotes NR⁴,
R⁴ denotes H or methyl,
R⁵ denotes methyl,
R^{2'}, R^{2"}, R³ denote H,
R^{1'}, R^{1"} denote H,
R¹ denotes H, Hal or methyl,
R^{1""} denotes H, Hal, methyl, ethyl, n-propyl, 2-propyl, butyl, isobutyl, sec-butyl, tert-butyl, methoxy, CHal₃, CF₃, OH, OCH₂CH₂OH, SCH₂CH₃, NHCH₃, N(CH₃)₂, CN, COOH, COOCH₃, SO₂OH, OCHal₃, OCF₃, NHCOA, NHCOAr, NHCON(R⁶)₂, NHCONHA, NHCONHAr, where A and R⁶ are H, cyclopentyl, cyclohexyl, n-propyl, 2-propyl, ethyl, sec-butyl or tert-butyl and Ar is thio-phen-2- or -3-yl, 3,5-dimethylisoxazol-4-yl, and two radicals R⁶, together with the amide nitrogen atom, can form a tetrahydro-pyrrole ring,
and pharmaceutically acceptable salts, solvates and stereoisomers thereof, including mixtures thereof in all ratios.

3. Process for the preparation of compounds of the formula AV and physiologically acceptable salts, solvates and stereoisomers thereof, **characterised in that**
a compound of the formula VIII 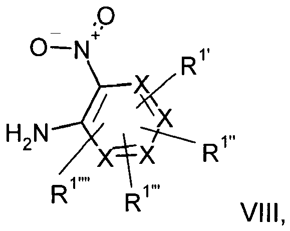 in which all radicals have the meanings indicated above, is reacted with a compound of the formula VII 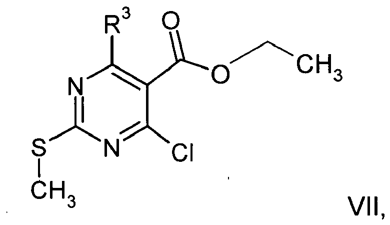 in which all radicals have the meanings indicated above, to give a compound of the formula VI 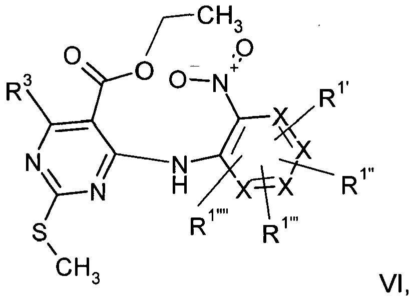 which is reduced to give a compound of the formula V 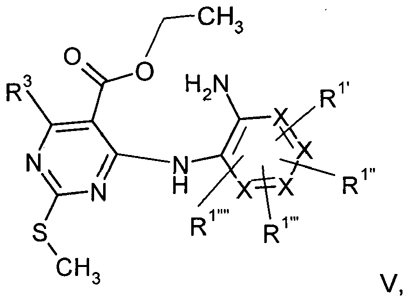 which is then saponified in the next step to give a compound of the formula IV 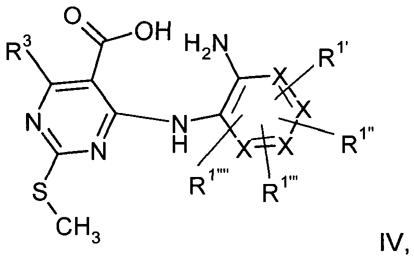 which is cyclised further to give a diazepinone of the formula III 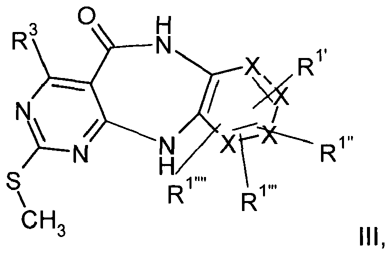 which, after an increase in the reactivity of the thioether, for example by oxidation to a sulfone, is substituted by a compound of the formula II 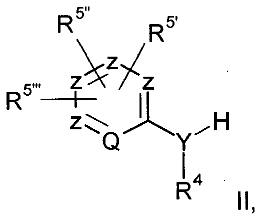 giving a compound of the formula Ib 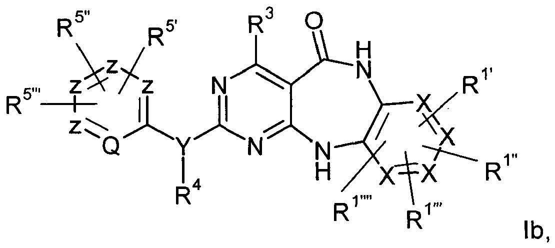 which is finally, if the radicals R^{2'}, R^{2"} have a meaning other than H, converted into a compound of the formula AV, and a base or acid of the formula AV is optionally converted into one of its salts.

4. Compounds according to one or more of Claims 1 to 3 and/or physiologically acceptable salts, solvates and stereoisomers thereof, including mixtures thereof in all ratios, for use as medicaments.

5. Medicaments comprising at least one compound according to one or more of Claims 1 to 3 and/or physiologically acceptable salts, solvates and stereoisomers thereof, including mixtures thereof in all ratios, and optionally excipients and/or adjuvants.

6. Medicaments comprising at least one compound according to one or more of Claims 1 to 3 and/or physiologically acceptable salts, solvates and stereoisomers thereof, including mixtures thereof in all ratios, and at least one further medicament active compound.

7. Set (kit) consisting of separate packs of
a) an effective amount of a compound according to one or more of Claims 1 to 3 and/or physiologically acceptable salts, solvates and stereoisomers thereof, including mixtures thereof in all ratios, and
b) an effective amount of a further medicament active compound.

8. Compounds according to one or more of Claims 1 to 3 and physiologically acceptable salts, solvates and stereoisomers thereof, including mixtures thereof in all ratios, for use as activators or inhibitors of kinases, in particular tyrosine kinases.

9. Compounds according to one or more of Claims 1 to 3 and physiologically acceptable salts, solvates and stereoisomers thereof, including mixtures thereof in all ratios, for use as inhibitors of the serine/threonine kinase PDK1.

10. Use of compounds according to one or more of Claims 1 to 3 and/or physiologically acceptable salts, solvates and stereoisomers thereof, including mixtures thereof in all ratios, for the preparation of a medicament for the prophylaxis or treatment of diseases in which inhibition of the serine/ threonine kinase PDK1 results in an improvement in the clinical picture.

11. Use of compounds according to one or more of Claims 1 to 3 and/or physiologically acceptable salts, solvates and stereoisomers thereof, including mixtures thereof in all ratios, for the preparation of a medicament for the prophylaxis or treatment of cancer, tumour growth, tumour angiogenesis, arteriosclerosis, diabetic retinopathy and inflammatory diseases.

12. Use of compounds according to one or more of Claims 1 to 3 and/or physiologically acceptable salts, solvates and stereoisomers thereof, including mixtures thereof in all ratios, for the preparation of a medicament for the prophylaxis or treatment of breast cancer, prostate cancer, colorectal cancer, small-cell lung cancer, non-small-cell lung cancer, multiple myeloma and renal-cell carcinoma, glioblastoma and endometrial carcinoma.

13. Compound according to Claim 1, selected from the group consisting of 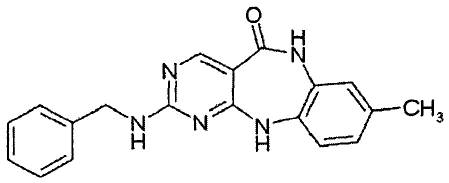 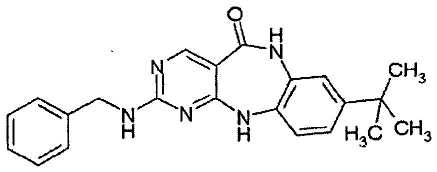 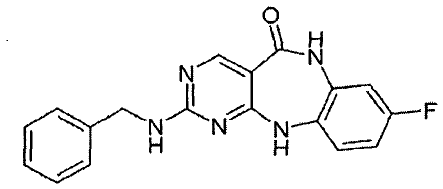 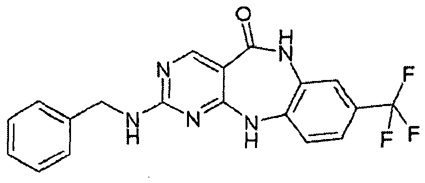 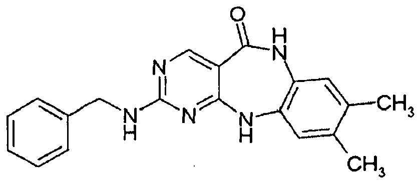 and pharmaceutically acceptable salts, solvates and stereoisomers thereof, including mixtures thereof in all ratios.

## Revendications

1. Composés de formule AV 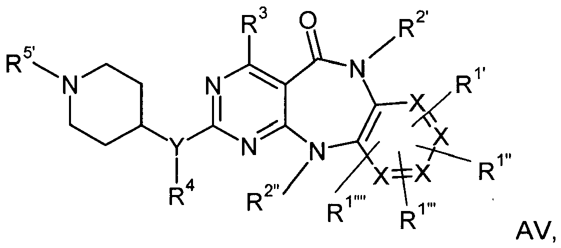 dans laquelle
R^{1'}, R^{1"}, R^{1'"}, R^{1""}, R³, R⁴, R^{5'} désignent chacun, indépendamment les uns des autres, H, A, R⁶, Ar, OR⁶, SR⁶, OAr, SAr, N(R⁶)₂, NHAr, Hal, NO₂, CN, (CH₂)ₘCOOR⁶, (CH₂)ₘCOOAr, (CH₂)ₘCON(R⁶)₂, (CH₂)ₘCONAAr, COA, COR⁶, COAr, S(O)ₘA, S(O)ₘAr, NACOA, NACOAr, NASO₂A, NASO₂Ar, NHCOA, NHCOAr, NHCON(R⁶)₂, NHCONHA, NHCONHAr, SO₂N(R⁶)₂, SO₂NAAr, M(CH₂)ₙN(R⁶)₂, M(CH₂)ₙNAR⁶, M(CH₂)ₙNA₂, M(CH₂)ₙ(R⁶)ₙ, M(CH₂)ₙ(R⁶)ₙ, M(CH₂)ₙ(R⁶)ₙ, M(**C**H₂)ₙ(R⁶)ₙ, M(CH₂)ₙ-oxopipérazine, M(CH₂)ₙ-oxomorpholine, M(CH₂)ₙ-oxopyrrolidine, M(CH₂)ₙC(CH₃)ₙ(CH₂)ₙN(R⁶)₂, M(CH₂)ₙM(R⁶)ₙSOₘA, M(CH₂)ₙM(R⁶)ₙSOₘM(R⁶)ₙ, M(CH₂)ₙM(R⁶)ₙSOₘAr, (CH₂)ₙM(R⁶)ₙSOₘA, (CH₂)ₙM(R⁶)ₙSOₘM(R⁶)ₙ, (CH₂)ₙM(R⁶)ₙSOₘAr, M(CH₂)ₙSOₘA, M(CH₂)ₙSOₘN(R⁶)ₙA, M(CH₂)ₙSOₘAr, (CH₂)ₙSOₘA, (CH₂)ₙSOₘM(R⁶)ₙ, (CH₂)ₙSOₘAr, où deux radicaux adjacents R^{1'}, R^{1"}, R^{1'"} ou R^{1""} peuvent former un carbo- ou hétérocycle de cinq ou six chaînons, saturé ou insaturé, qui est éventuellement mono- ou disubstitué par M l'un avec l'autre,
R^{2'}, R^{2"} désignent chacun, indépendamment les uns des autres, R⁶,
R⁶ désigne H, Hal, OH, CN, NH₂, NO₂, SO₂, alkyle non ramifié ou ramifié ayant 1-4 atomes de C, où un groupement CH₂ peut être remplacé par un atome de O ou S et/ou par un groupement NH, NA, CONH, NHCO ou -CH=CH- et/ou, de plus, 1-4 atomes de H peuvent être remplacés par Hal, et où un groupement CH₃ peut être remplacé par Hal, OH, CN, NH₂, NHR₇, NR⁷₂, NO₂ ou SO₂, où R⁷ = méthyle ou éthyle,
où deux radicaux R⁶, conjointement avec l'atome auquel ils sont fixés, peuvent former un carbo- ou hétérocycle de cinq ou six chaînons, saturé ou insaturé,
n désigne 0, 1, 2, 3, 4 ou 5,
m désigne 0, 1 ou 2,
A désigne alkyle non ramifié, ramifié ou cyclique ayant 1-14 atomes de C, où un ou deux groupements CH₂ peuvent être remplacés par un atome de O ou S et/ou par un groupement NH, CONH, NHCO, CO ou -CH=CH- et/ou, de plus, 1-7 atomes de H peuvent être remplacés par Hal, et où un ou deux groupements CH₃ peuvent être remplacés par R⁶,
Ar désigne un homo- ou hétérocycle mono- ou bicyclique aromatique ayant de 1 à 4 atomes de N, O et/ou S et de 5 à 10 atomes de squelette, qui peut être non substitué ou mono-, diou trisubstitué par oxygène du carbonyle, Hal, A, OH, OA, NH₂, NHA, NA₂, NO₂, CN, OCN, SCN, COOH, COOA, CONH₂, CONHA, CONA₂, NHCOA, NHCONH₂, NHSO₂A, CHO, COA, SO₂NH₂ et/ou S(O)ₘA,
Hal désigne F, CI, Br ou I,
X est CR¹, où R¹ est représentatif de l'un des radicaux R¹, R^{1"}, R^{1'"} ou R^{1""},
Y désigne NR⁴, O ou S, et
M désigne NH, O, S,
et les sels, solvats et stéréoisomères pharmaceutiquement acceptables de ceux-ci, y compris des mélanges de ceux-ci en toutes proportions.

2. Composés selon la revendication 1, dans lesquels dans la sous-formule Ac
R^{5'} désigne méthyle, dans la sous-formule Ad
R³ désigne H,
dans la sous-formule Ae
R^{2'}, R^{2"} désignent H,
dans la sous-formule Af
Y désigne NR⁴,
R⁴ désigne H ou méthyle,
dans la sous-formule Ag
R^{1'}, R^{1"} désignent H,
R^{1"'} désigne H, Hal ou méthyle,
R^{1"'} désigne H, Hal, méthyle, éthyle, n-propyle, 2-propyle, butyle, isobutyle, sec-butyle, tertio-butyle, méthoxy, CHaI₃, CF₃, OH, OCH₂CH₂OH, SCH₂CH₃, NHCH₃, N(CH₃)₂, CN, COOH, COOCH₃, SO₂OH, OCHaI₃, OCF₃, NHCOA, NHCOAr, NHCON(R⁶)₂, NHCONHA, NHCONHAr, où A et R⁶ sont H, cyclopentyle, cyclohexyle, n-propyle, 2-propyle, éthyle, sec-butyle ou tertio-butyle et Ar est thiophén-2- ou -3-yle, 3,5-diméthylisoxazol-4-yle, et deux radicaux R⁶, conjointement avec l'atome d'azote d'amide, peuvent former un cycle tétrahydropyrrole,
dans la sous-formule Ah
Y désigne NR⁴,
R⁴ désigne H ou méthyle,
R^{5'} désigne méthyle,
R^{2'}, R^{2"}, R³ désignent H,
R^{1'}, R^{1"} désignent H,
R^{1"'} désigne H, Hal ou méthyle,
R^{1"'} désigne H, Hal, méthyle, éthyle, n-propyle, 2-propyle, butyle, isobutyle, sec-butyle, tertio-butyle, méthoxy, CHaI₃, CF₃, OH, OCH₂CH₂OH, SCH₂CH₃, NHCH₃, N(CH₃)₂, CN, COOH, COOCH₃, SO₂OH, OCHaI₃, OCF₃, NHCOA, NHCOAr, NHCON(R⁶)₂, NHCONHA, NHCONHAr, où A et R⁶ sont H, cyclopentyle, cyclohexyle, n-propyle, 2-propyle, éthyle, sec-butyle ou tertio-butyle et Ar est thiophén-2- ou -3-yle, 3,5-diméthylisoxazol-4-yle, et deux radicaux R⁶, conjointement avec l'atome d'azote d'amide, peuvent former un cycle tétrahydro-pyrrole,
et les sels, solvats et stéréoisomères pharmaceutiquement acceptables de ceux-ci, y compris des mélanges de ceux-ci en toutes proportions.

3. Procédé de préparation de composés de formule AV et de sels, solvats et stéréoisomères physiologiquement acceptables de ceux-ci, **caractérisé en ce que**
un composé de formule VIII 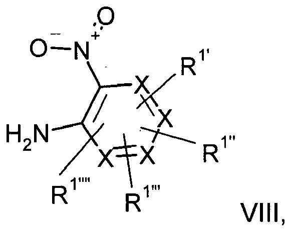 dans laquelle tous les radicaux revêtent les significations indiquées ci-dessus, est réagi avec un composé de formule VII 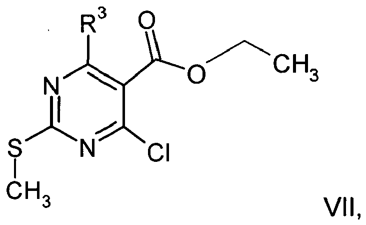 dans laquelle tous les radicaux revêtent les significations indiquées ci-dessus, pour donner un composé de formule VI 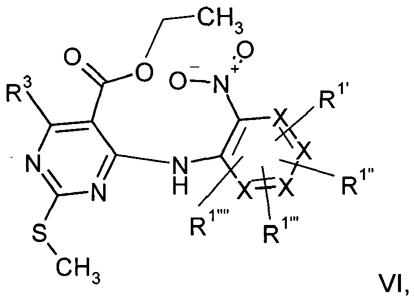 qui est réduit pour donner un composé de formule V 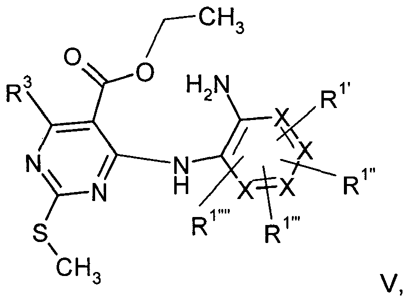 qui est ensuite saponifié dans l'étape suivante pour donner un composé de formule IV 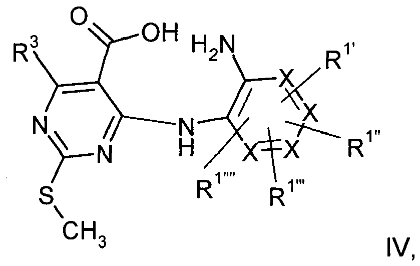 qui est cyclisé davantage pour donner une diazépinone de formule III 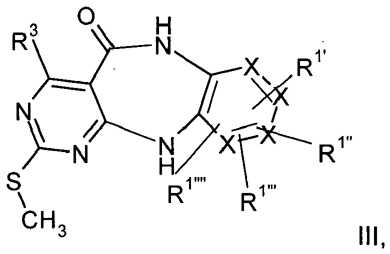 qui, après une augmentation de la réactivité du thioéther, par exemple par oxydation en une sulfone, est substituée par un composé de formule II 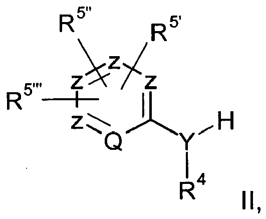 donnant un composé de formule Ib 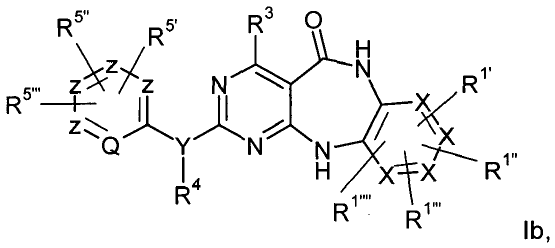 qui est finalement, si les radicaux R^{2'}, R^{2"} revêtent une signification autre que H, converti en un composé de formule AV, et une base ou un acide de formule AV est éventuellement converti(e) en l'un de ses sels.

4. Composés selon l'une ou plusieurs parmi les revendications 1 à 3 et/ou des sels, solvats et stéréoisomères physiologiquement acceptables de ceux-ci, y compris des mélanges de ceux-ci en toutes proportions, pour une utilisation comme médicaments.

5. Médicaments comprenant au moins un composé selon l'une ou plusieurs parmi les revendications 1 à 3 et/ou des sels, solvats et stéréoisomères physiologiquement acceptables de celui-ci, y compris des mélanges de ceux-ci en toutes proportions, et éventuellement des excipients et/ou des adjuvants.

6. Médicaments comprenant au moins un composé selon l'une ou plusieurs parmi les revendications 1 à 3 et/ou des sels, solvats et stéréoisomères physiologiquement acceptables de celui-ci, y compris des mélanges de ceux-ci en toutes proportions, et au moins un autre composé actif médicamenteux.

7. Ensemble (kit) constitué de conditionnements séparés
a) d'une quantité efficace d'un composé selon l'une ou plusieurs parmi les revendications 1 à 3 et/ou de sels, solvats et stéréoisomères physiologiquement acceptables de celui-ci, y compris des mélanges de ceux-ci en toutes proportions, et
b) d'une quantité efficace d'un autre composé actif médicamenteux.

8. Composés selon l'une ou plusieurs parmi les revendications 1 à 3 et des sels, solvats et stéréoisomères physiologiquement acceptables de ceux-ci, y compris des mélanges de ceux-ci en toutes proportions, pour une utilisation comme activateurs ou inhibiteurs de kinases, en particulier de tyrosine kinases.

9. Composés selon l'une ou plusieurs parmi les revendications 1 à 3 et des sels, solvats et stéréoisomères physiologiquement acceptables de ceux-ci, y compris des mélanges de ceux-ci en toutes proportions, pour une utilisation comme inhibiteurs de la sérine/thréonine kinase PDK1.

10. Utilisation de composés selon l'une ou plusieurs parmi les revendications 1 à 3 et/ou de sels, solvats et stéréoisomères physiologiquement acceptables de ceux-ci, y compris des mélanges de ceux-ci en toutes proportions, pour la préparation d'un médicament destiné à la prophylaxie ou au traitement de maladies dans lesquelles l'inhibition de la sérine/thréonine kinase PDK1 entraîne une amélioration du tableau clinique.

11. Utilisation de composés selon l'une ou plusieurs parmi les revendications 1 à 3 et/ou de sels, solvats et stéréoisomères physiologiquement acceptables de ceux-ci, y compris des mélanges de ceux-ci en toutes proportions, pour la préparation d'un médicament destiné à la prophylaxie ou au traitement de cancers, de la croissance tumorale, de l'angiogenèse de tumeurs, de l'artériosclérose, de la rétinopathie diabétique et de maladies inflammatoires.

12. Utilisation de composés selon l'une ou plusieurs parmi les revendications 1 à 3 et/ou de sels, solvats et stéréoisomères physiologiquement acceptables de ceux-ci, y compris des mélanges de ceux-ci en toutes proportions, pour la préparation d'un médicament destiné à la prophylaxie ou au traitement du cancer du sein, du cancer de la prostate, du cancer colorectal, du cancer du poumon à petites cellules, du cancer du poumon non à petites cellules, du myélome multiple et de l'hypernéphrome, du glioblastome et du carcinome de l'endomètre.

13. Composé selon la revendication 1, choisi dans le groupe constitué par 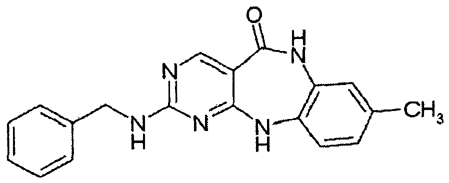 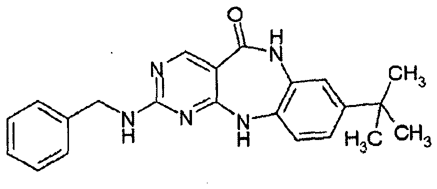 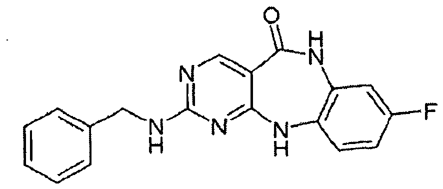 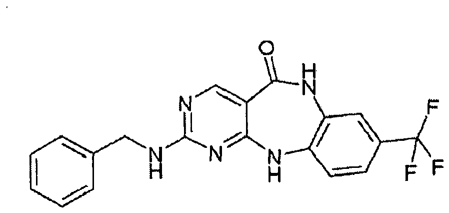 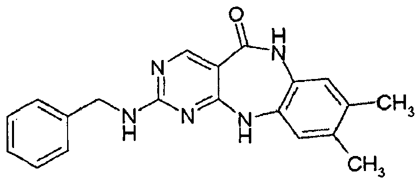 et les sels, solvats et stéréoisomères pharmaceutiquement acceptables de ceux-ci, y compris des mélanges de ceux-ci en toutes proportions.
